# EUROPEAN PATENT SPECIFICATION

(11) **EP 0 313 578 B1**
(45) Date of publication and mention of the grant of the patent: **28.08.1996**
(21) Application number: 87905023.5
(22) Date of filing: 30.06.1987
(51) Int. Cl.: C07K 14/51, C12N 15/12, A61K 38/18

(54) **NOVEL OSTEOINDUCTIVE COMPOSITIONS**
OSTEOINDUKTIVE MITTEL
NOUVELLES COMPOSITIONS OSTEOINDUCTIVES

(30) Priority: 01.07.1986 US 880776; 17.12.1986 US 943332; 20.03.1987 US 28285; 26.03.1987 US 31346
(43) Date of publication of application: 03.05.1989
(60) Divisional application: 95111771.2
(73) Proprietor: GENETICS INSTITUTE, INC., Cambridge, Massachusetts 02140 (US)
(72) Inventor: WANG, Elizabeth, A., Carlisle, MA 01741 (US); WOZNEY, John, M., Hudson, MA 01749 (US); ROSEN, Vicki, A., Boston, MA 02116 (US)
(74) Representative: VOSSIUS & PARTNER
(86) International application number: US8701537
(87) International publication number: WO8800205

(56) References cited:
- EP-A- 0 121 976
- EP-A- 0 128 041
- EP-A- 0 148 155
- EP-A- 0 212 474
- WO-A-85/04173
- US-A- 4 455 256
- US-A- 4 563 350
- US-A- 4 619 989
- PROCEEDINGS OF THE NATL. ACADEMY OF SCIENCES USA, vol. 81, January 1984, Washington, DC (US); URIST, pp. 371-375
- SCIENCE, vol. 220, 13 May 1983, Washington, DC (US); URIST, pp. 680-686
- PROCEEDINGS OF THE NATL. ACADEMY OF SCIENCES USA, vol. 80, November 1983, Washington, DC (US); SAMPATH et al., pp. 6591-6595
- PROCEEDINGS OF THE NATL. ACADEMY OF SCIENCES USA, vol. 78, November 1981, Washington, DC (US); SUGGS et al., pp. 6613-6617

## Description

The present invention relates to novel proteins, processes for obtaining them and genes encoding them. These proteins are capable of inducing cartilage and bone formation.

### Background

Bone is a highly specialized tissue characterized by an extensive matrix structure formed of fibrous bundles of the protein collagen, and proteoglycans, noncollagenous proteins, lipids and acidic proteins. The processes of bone formation and renewal/repair of bone tissue, which occur continuously throughout life, are performed by specialized cells. Normal embryonic long bone development is preceded by formation of a cartilage model. Bone growth is presumably mediated by "osteoblasts" (bone-forming cells), while remodeling of bone is apparently accomplished by the joint activities of bone-resorbing cells, called "osteoclasts" and osteoblasts. A variety of osteogenic, cartilage-inducing and bone inducing factors have been described. See, e.g. European patent applications 148,155 and 169,016 for discussions thereof.

### Brief Description of the Invention

The present invention provides novel proteins in purified form and genes encoding them. Specifically, two of the novel proteins are designated BMP-2 Class I (or BMP-2), and BMP-2 Class II (or BMP-4) wherein BMP is bone morphogenic protein. These proteins are characterized by peptide sequences the same as or substantially homologous to amino acid sequences illustrated in Tables II, III and IV below. They are capable of inducing bone formation at a predetermined site. These bone inductive factors are further characterized by biochemical and biological characteristics including activity at a concentration of 10 to 1000ng/gram of bone in an in vivo rat bone formation assay described below. Proteins of this invention may be encoded by the DNA sequences depicted in the Tables or by sequences capable of hybridizing thereto and coding for polypeptides with bone growth factor biological properties or other variously modified sequences demonstrating such properties.

One of the proteins of the invention is designated BMP-2 Class I (or BMP-2). It is characterized by at least a portion of a peptide sequence the same or substantially the same as that of amino acid #1 through amino acid #396 of Table III which represents the cDNA hBMP-2 Class I. This peptide sequence is encoded by the same or substantially the same DNA sequence, as depicted in nucleotide #356 through nucleotide #1543 of Table III. The human peptide sequence identified in Table III is 396 amino acids in length. hBMP-2 or related bone inductive proteins may also be characterized by at least a portion of this peptide sequence. hBMP-2 Class I is further characterized by the ability to induce bone formation.

The homologous bovine bone inductive protein of the invention designated bBMP-2 Class I (or bBMP-2), has a DNA sequence identified in Table II below which represents the genomic sequence. This bovine DNA sequence has a prospective 129 amino acid coding sequence followed by approximately 205 nucleotides (a presumptive 3' non-coding sequence). bBMP-2, Class I is further characterized by the ability to induce bone formation. A further bone inductive protein composition of the invention is designated BMP-2 Class II or BMP-4. The human protein hBMP-2 Class II (or hBMP-4) is characterized by at least a portion of the same or substantially the same peptide sequence between amido acid #1 through amino acid #408 of Table IV, which represents the cDNA of hBMP-2 Class II. This peptide sequence is encoded by at least a portion of the same or substantially the same DNA sequence as depicted in nucleotide #403 through nucleotide #1626 of Table IV. This factor is further characterized by the ability to induce bone formation.

Another aspect of the invention provides pharmaceutical compositions containing a therapeutically effective amount of one or more bone growth factor polypeptides according to the invention in a pharmaceutically acceptable vehicle. These compositions may further include other therapeutically useful agents. They may also include an appropriate matrix for delivering the proteins to the site of the bone defect and for providing a structure for bone growth. These compositions may be employed in methods for treating a number of bone defects and periodontal disease. These methods, according to the invention, entail administering to a patient needing such bone formation an effective amount of at least one of the novel proteins BMP-2 Class I and BMP-2 Class-II as described herein.

Still a further aspect of the invention are DNA sequences coding on expression for a human or bovine polypeptide having the ability to induce bone formation. Such sequences include the sequence of nucleotides in a 5' to 3' direction illustrated in Tables II, III and IV. Alternatively, a DNA sequence which hybridizes under stringent conditions with the DNA sequences of Tables II, III and IV or a DNA sequence which hybridizes under non-stringent conditions with the illustrated DNA sequences and which codes on expression for a protein having at least one bone growth factor biological property are included in the present invention. Finally, allelic or other variations of the sequences of Tables II, III and IV, whether such nucleotide changes result in changes in the peptide sequence or not, are also included in the present invention.

Still a further aspect of the invention is a vector containing a DNA sequence as described above in operative association with an expression control sequence. Such vector may be employed in a novel process for producing a bone growth factor polypeptide in which a cell line transformed with a DNA sequence encoding expression of a bone growth factor polypeptide in operative association with an expression control sequence therefor, is cultured. This claimed process may employ a number of known cells as host cells for expression of the polypeptide. Presently preferred cell lines are mammalian cell lines and bacterial cells.

Other aspects and advantages of the present invention will be apparent upon consideration of the following detailed description and preferred embodiments thereof.

### Detailed Description of the Invention

The proteins of the present invention are characterized by amino acid sequences or portions thereof the same as or substantially homologous to the sequences shown in Tables II, III and IV. These proteins are also characterized by the ability to induce bone formation.

The bone growth factors provided herein also include factors encoded by the sequences similar to those of Tables II, III and IV, but into which modifications are naturally provided (e.g. allelic variations in the nucleotide sequence which may result in amino acid changes in the polypeptide) or deliberately engineered. For example, synthetic polypeptides may wholly or partially duplicate continuous sequences of the amino acid residues of Tables II, III and IV. These sequences, by virtue of sharing primary, secondary, or tertiary structural and conformational characteristics with bone growth factor polypeptides of Tables II, III and IV may possess bone growth factor biological properties in common therewith. Thus, they may be employed as biologically active substitutes for naturally-occurring bone growth factor polypeptides in therapeutic processes.

Other specific mutations of the sequences of the bone growth factors described herein involve modifications of one or both of the glycosylation sites. The absence of glycosylation or only partial glycosylation results from amino acid substitution or deletion at one or both of the asparagine-linked glycosylation recognition sites present in the sequences of the bone growth factors shown in Tables II, III and IV. The asparagine-linked glycosylation recognition sites comprise tripeptide sequences which are specifically recognized by appropriate cellular glycosylation enzymes. These tripeptide sequences are either asparagine-X-threonine or asparagine-X-serine, where X is usually any amino acid. A variety of amino acid substitutions or deletions at one or both of the first or third amino acid positions of a glycosylation recognition site (and/or amino acid deletion at the second postion) results in non-glycosylation at the modified tripeptide sequence.

The present invention also encompasses the novel DNA sequences, free of association with DNA sequences encoding other proteinaceous materials, and coding on expression for bone growth factors. These DNA sequences include those depicted in Tables II, III and IV in a 5' to 3' direction and those sequences which hybridize under stringent hybridization conditions [see, T. Maniatis et al, Molecular Cloning (A Laboratory Manual), Cold Spring Harbor Laboratory (1982), pages 387 to 389] to the DNA sequences of Tables II, III and IV.

DNA sequences which hybridize to the sequences of Tables II, III and IV under relaxed hybridization conditions and which code on expression for bone growth factors having bone growth factor biological properties also encode bone growth factors of the invention. For example, a DNA sequence which shares regions of significant homology, e.g., sites of glycosylation or disulfide linkages, with the sequences of Tables II, III and IV and encodes a bone growth factor having one or more bone growth factor biological properties clearly encodes a member of this novel family of growth factors, even if such a DNA sequence would not stringently hybridize to the sequence of Tables II, III and IV.

Similarly, DNA sequences which code for bone growth factor polypeptides coded for by the sequences of Tables II, III and IV, but which differ in codon sequence due to the degeneracies of the genetic code or allelic variations (naturally-occurring base changes in the species population which may or may not result in an amino acid change) also encode the novel growth factors described herein. Variations in the DNA sequences of Tables II, III and IV which are caused by point mutations or by induced modifications to enhance the activity, half-life or production of the polypeptides encoded thereby are also encompassed in the invention.

Another aspect of the present invention provides a novel method for producing the novel osteoinductive factors. The method of the present invention involves culturing a suitable cell or cell line, which has been transformed with a DNA sequence coding on expression for a novel bone growth factor polypeptide of the invention, under the control of known regulatory sequences.. Suitable cells or cell lines may be mammalian cells, such as Chinese hamster ovary (CHo) cells. The selection of suitable mammalian host cells and methods for transformation, culture, amplification, screening and product production and purification are known in the art. See, e.g., Gething and Sambrook, Nature, 293:620-625 (1981), or alternatively, Kaufman et al, Mol. Cell. Biol., 5(7):1750-1759 (1985) or Howley et al, U.S. Patent 4,419,446. Another suitable mammalian cell line, which is described in the accompanying examples, is the monkey COS-1 cell line. A similarly useful mammalian cell line is the CV-1 cell line.

Bacterial cells are suitable hosts. For example, the various strains of E. coli (e.g., HB101, MC1061) are well-known as host cells in the field of biotechnology. Various strains of B. subtilis, Pseudomonas, other bacilli and the like may also be employed in this method.

Many strains of yeast cells known to those skilled in the art are also available as host cells for expression of the polypeptides of the present invention. Additionally, where desired, insect cells may be utilized as host'cells in the method of the present invention. See, e.g. Miller et al, Genetic Engineering, 8:277-298 (Plenum Press 1986) and references cited therein.

Another aspect of the present invention provides vectors for use in the method of expression of these novel osteoinductive polypeptides. Preferably the vectors contain the full novel DNA sequences described above which code for the novel factors of the invention. Additionally the vectors also contain appropriate expression control sequences permitting expression of the bone inductive protein sequences. Alternatively, vectors incorporating modified sequences as described above are also embodiments of the present invention and useful in the production of the bone inductive proteins. The vectors may be employed in the method of transforming cell lines and contain selected regulatory sequences in operative association with the DNA coding sequences of the invention which are capable of directing the replication and expression thereof in selected host cells. Useful regulatory sequences for such vectors are known to one of skill in the art and may be selected depending upon the selected host cells. Such selection is routine and does not form part of the present invention.

A protein of the present invention, which induces bone growth in circumstances where bone is not normally formed, has application in the healing of bone fractures. An osteogenic preparation employing one or more of the proteins of the invention may have prophylactic use in closed as well as open fracture reduction and also in the improved fixation of artificial joints. De novo bone formation induced by an osteogenic agent contributes to the repair of congenital, trauma induced, or oncologic resection induced craniofacial defects, and also is useful in cosmetic plastic surgery. An osteogenic factor of the invention may be valuable in the treatment of periodontal disease, and in other tooth repair processes. Such agents may provide an environment to attract bone-forming cells, stimulate growth of bone-forming cells or induce differentiation of progenitors of bone-forming cells. Of course, the proteins of the invention may have other therapeutic uses.

A further aspect of the invention is a therapeutic method and composition for repairing fractures and other conditions related to bone defects or periodontal diseases. Such a composition comprises a therapeutically effective amount of at least one of the bone inductive factor proteins of the invention. The bone inductive factors according to the present invention may be present in a therapeutic compositon in admixture with a pharmaceutically acceptable vehicle or matrix. Further therapeutic methods and compositions of the invention comprise a therapeutic amount of a bone inductive factor of the invention with a therapeutic amount of at least one of the other bone inductive factors of the invention. Additionally, the proteins according to the present invention or a combination of the proteins of the present invention may be co-administered with one or more different osteoinductive factors with which they may interact. Further, the bone inductive proteins may be combined with other agents beneficial to the treatment of the bone defect in question. Such agents include, but are not limited to various growth factors. The preparation of such physiologically acceptable protein compositions, having due regard to pH, isotonicity, stability and the like, is within the skill of the art.

In particular, BMP-2 Class I may be used individually in a pharmaceutical composition. BMP-2 Class I may also be used in combination with one or more of the other proteins of the invention. BMP-2 Class I may be combined with BMP-2 Class II. It may also be combined with BMP-3. Further BMP-2 Class I may be combined with BMP-2 Class II and BMP-3.

BMP-2 Class II may be used individually in pharmaceutical composition. In addition, it may be used in combination with other proteins as identified above. Further it may be used in combination with BMP-3.

The therapeutic method includes locally administering the composition as an implant or device. When administered, the therapeutic composition for use in this invention is, of course, in a pyrogen-free, physiologically acceptable form. Further, the composition may desirably be encapsulated or injected in a viscous form for delivery to the site of bone damage. Preferably, the bone growth inductive factor composition would include a matrix capable of delivering the bone inductive factor to the site of bone damage, providing a structure for the developing bone and cartilage and optimally capable of being resorbed into the body. Such matrices may be formed of other materials presently in use for other implanted medical applications.

The choice of material is based on, for example, biocompatibility, biodegradability, mechanical properties, cosmetic appearance and interface properties. Similarly, the application of the osteoinductive factors will define the appropriate formulation. Potential matrices for the osteoinductive. factors may be biodegradable and chemically defined, such as, but not limited to calcium sulfate, tricalciumphosphate, hydroxyapatite, polylactic acid, polyanhydrides; biodegradable and biologically well defined, such as bone or dermal collagen, other pure proteins or extracellular matrix components; nonbiodegradable and chemically defined, such as sintered hydroxyapatite, bioglass, aluminates, or other ceramics; or combinations of any of the above mentioned types of material, such as polylactic acid and hydroxyapatite or collagen and tricalciumphosphate. The bioceramics might also be altered in composition, such as in calcium-aluminate-phosphate and processing to alter for example, pore size, particle size, particle shape, and biodegradability.

The dosage regimen will be determined by the attending physician considering various factors which modify the action of such a growth factor, e.g. amount of bone weight desired to be formed, the site of bone damage, the condition of the damaged bone, the patient's age, sex, and diet, the severity of any infection, time of administration and other clinical factors. The dosage may vary with the type of matrix used in the reconstitution and the composition of BMP's. The addition of other known growth factors, such as IGF 1 (insulin like growth factor 1), to the final composition, may also effect the dosage. Generally, the dosage regimen should be in the range of approximately 10 to 10⁶ nanograms of protein per gram of bone weight desired. Progress can be monitored by periodic assessment of bone growth and/or repair, e.g. x-rays. Such therapeutic compositions are also presently valuable for veterinary applications due to the lack of species specificity in bone inductive factors. Particularly domestic animals and thoroughbred horses in addition to humans are desired patients for such treatment with the bone inductive factors of the present invention.

The following examples illustrate practice of the present invention in recovering and characterizing the bovine proteins and employing them to recover the human proteins, obtaining the human proteins and in expressing the proteins via recombinant techniques.

### EXAMPLE I

### Isolation of Bovine Bone Inductive Factor

Ground bovine bone powder (20-120 mesh, Helitrex) is prepared according to the procedures of M. R. Urist et al., Proc. Natl Acad. Sci USA, 70:3511 (1973) with elimination of some extraction steps as identified below. Ten kgs of the ground powder is demineralized in succesive changes of 0.6N HCl at 4°C over a 48 hour period with vigorous stirring. The resulting suspension is extracted for 16 hours at 4°C with 50 liters of 2M CaCl₂ and 10mM ethylenediamine-tetraacetic acid [EDTA], and followed by extraction for 4 hours in 50 liters of 0.5M EDTA. The residue is washed three times with distilled water before its resuspension in 20 liters of 4M guanidine hydrochloride [GuCl], 20mM Tris (pH 7.4), 1 mM N-ethylmaleimide, 1mM iodoacetamide, 1mM phenylmethylsulfonyl fluoride as described in Clin. Orthop. Rel. Res., 171: 213 (1982). After 16 to 20 hours the supernatant is removed and replaced with another 10 liters of GuCl buffer. The residue is extracted for another 24 hours.

The crude GuCl extracts are combined, concentrated approximately 20 times on a Pellicon apparatus with a 10,000 molecular weight cut-off membrane, and then dialyzed in 50mM Tris, 0.1M NaCl, 6M urea (pH7.2), the starting buffer for the first column. After extensive dialysis the protein is loaded on a 4 liter DEAE cellulose column and the unbound fractions are collected.

The unbound fractions are concentrated and dialyzed against 50mM NaAc, 50mM NaCl (pH 4.6) in 6M urea. The unbound fractions are applied to a carboxymethyl cellulose column. Protein not bound to the column is removed by extensive washing with starting buffer, and the bone inductive factor containing material desorbed from the column by 50mM NaAc, 0.25mM NaCl, 6M urea (pH 4.6). The protein from this step elution is concentrated 20- to 40- fold, then diluted 5 times with 80mM KPO₄, 6M urea (pH6.0). The pH of the solution is adjusted to 6.0 with 500mM K₂HPO₄. The sample is applied to an hydroxylapatite column (LKB) equilibrated in 80mM KPO₄, 6M urea (pH6.0) and all unbound protein is removed by washing the column with the same buffer. Bone inductive factor activity is eluted with 100mM KPO₄ (pH7.4) and 6M urea.

The protein is concentrated approximately 10 times, and solid NaCl added to a final concentration of 0.15M. This material is applied to a heparin - Sepharose column equilibrated in 50mM KPO₄, 150mM NaCl, 6M urea (pH7.4). After extensive washing of the column with starting buffer, a protein with bone inductive factor activity is eluted by 50mM KPO₄, 700mM NaCl, 6M urea (pH7.4). This fraction is concentrated to a minimum volume, and 0.4ml aliquots are applied to Superose 6 and Superose 12 columns connected in series, equilibrated with 4M GuCl, 20mM Tris (pH7.2) and the columns developed at a flow rate of 0.25ml/min. The protein demonstrating bone inductive factor activity has a relative migration corresponding to approximately 30,000 dalton protein.

The above fractions are pooled, dialyzed against 50mM NaAc, 6M urea (pH4.6), and applied to a Pharmacia MonoS HR column. The column is developed with a gradient to 1.0M NaCl, 50mM NaAc, 6M urea (pH4.6). Active fractions are pooled and brought to pH3.0 with 10% trifluoroacetic acid (TFA). The material is applied to a 0.46 x 25cm Vydac C4 column in 0.1% TFA and the column developed with a gradient to 90% acetonitrile, 0.1% TFA (31.5% acetonitrile, 0.1% TFA to 49.5% acetonitrile, 0.1% TFA in 60 minutes at lml per minute). Active material is eluted at approximately 40-44% acetonitrile. Aliquots of the appropriate fractions are iodinated by one of the following methods: P. J. McConahey et al, Int. Arch. Allergy, 29:185-189 (1966); A. E. Bolton et al, Biochem J., 133:529 (1973); and D. F. Bowen-Pope, J. Biol. Chem., 237:5161 (1982). The iodinated proteins present in these fractions are analyzed by SDS gel electrophoresis and urea Triton X 100 isoelectric focusing. At this stage, the bone inductive factor is estimated to be approximately 10-50% pure.

### EXAMPLE II

### Characterization of Bovine Bone Inductive Factor

### A. Molecular Weight

Approximately 20ug protein from Example I is lyophilized and redissolved in 1X SDS sample buffer. After 15 minutes of heating at 37°C, the sample is applied to a 15% SDS polyacrylamide gel and then electrophoresed with cooling. The molecular weight is determined relative to prestained molecular weight standards (Bethesda Research Labs). Immediately after completion, the gel lane containing bone inductive factor is sliced into 0.3cm pieces. Each piece is mashed and 1.4ml of 0.1% SDS is added. The samples are shaken gently overnight at room temperature to elute the protein. Each gel slice is desalted to prevent interference in the biological assay. The supernatant from each sample is acidified to pH 3.0 with 10% TFA, filtered through a 0.45 micron membrane and loaded on a 0.46cm x 5cm C4 Vydac column developed with a gradient of 0.1% TFA to 0.1% TFA, 90% CH₃CN. The appropriate bone inductive factor - containing fractions are pooled and reconstituted with 20mg rat matrix. In this gel system, the majority of bone inductive factor fractions have the mobility of a protein having a molecular weight of approximately 28,000 - 30,000 daltons.

### B. Isoelectric Focusing

The isoelectric point of bone inductive factor activity is determined in a denaturing isoelectric focusing system. The Triton X100 urea gel system (Hoeffer Scientific) is modified as follows: 1) 40% of the ampholytes used are Servalyte 3/10; 60% are Servalyte 7-9. 2) The catholyte used is 40mM NaOH. Approximately 20ug of protein from Example I is lyophilized, dissolved in sample buffer and applied to the isoelectrofocusing gel. The gel is run at 20 watts, 10°C for approximately 3 hours. At completion the lane containing bone inductive factor is sliced into 0.5 cm slices. Each piece is mashed in 1.0ml 6M urea, 5mM Tris (pH 7.8) and the samples agitated at room temperature. The samples are acidified, filtered, desalted and assayed as described above. The major portion of activity as determined in the assay described in Example III migrates in a manner consistent with a pI of 8.8 - 9.2.

### C. Subunit Characterization

The subunit composition of bone inductive factor is also determined. Pure bone inductive factor is isolated from a preparative 15% SDS gel as described above. A portion of the sample is then reduced with 5mM DTT in sample buffer and re-electrophoresed on a 15% SDS gel. The approximately 30kd protein yields two major bands at approximately 20kd and 18kd, as well as a minor band at 30kd. The broadness of the two bands indicates heterogeneity caused most probably by glycosylation, other post translational modification, proteolytic degradation or carbamylation.

### EXAMPLE III

### Biological Activity of Bone Inductive Factor

A rat bone formation assay according to the general procedure of Sampath and Reddi, Proc. Natl. Acad. Sci. U.S.A., 80:6591-6595 (1983) is used to evaluate the osteogenic activity of the bovine bone inductive factor of the present invention obtained in Example I. This assay can also be used to evaluate bone inductive factors of other species. The ethanol precipitation step is replaced by dialyzing the fraction to be assayed against water. The solution or suspension is then redissolved in a volatile solvent, e.g. 0.1 - 0.2 % TFA, and the resulting solution added to 20mg of rat matrix. This material is frozen and lyophilized and the resulting powder enclosed in #5 gelatin capsules. The capsules are implanted subcutaneously in the abdominal thoracic area of 21 - 49 day old male long Evans rats. The implants are removed after 7 - 14 days. Half of each implant is used for alkaline phosphatase analysis [See, A. H. Reddi et al., Proc. Natl.Acad.Sci., 69:1601 (1972)] and half is fixed and processed for histological analysis. Routinely, 1µm glycolmethacrylate sections are stained with Von Kossa and acid fuchsin to detect new bone mineral. Alkaline phosphatase, an enzyme produced by chondroblasts and osteoblasts in the process of matrix formation, is also measured. New cartilage and bone formation often correlates with alkaline phosphatase levels. Table I below illustrates the dose response of the rat matrix samples including a control not treated with bone inductive factor.

**TABLE 1**

| Protein* Implanted µg | Cartilage | Alk. Phos.u/l |
|---|---|---|
| 7.5 | 2 | Not done |
| 2.5 | 3 | 445.7 |
| 0.83 | 3 | 77.4 |
| 0.28 | 0 | 32.5 |
| 0.00 | 0 | 31.0 |

| | | |
|---|---|---|
| *At this stage the bone inductive factor is approximately 10-15% pure. | | |

The bone or cartilage formed is physically confined to the space occupied by the matrix. Samples are also analyzed by SDS gel electrophoresis and isoelectric focusing as described above, followed by autoradiography. Analysis reveals a correlation of activity with protein bands at 28 - 30kd and a pI 9.0. An extinction coefficient of 1 OD/mg-cm is used as an estimate for protein and approximating the purity of bone inductive factor in a particular fraction. In the in vivo rat bone formation assays on dilutions as described above, the protein is active in vivo at 10 to 200ng protein/gram bone to probably greater than 1µg protein/gram bone.

### EXAMPLE IV

### Bovine Bone Inductive Factor Protein Composition

The protein composition of Example IIA of molecular weight 28 - 30kd is reduced as described in Example IIC and digested with trypsin. Eight tryptic fragments are isolated by standard procedures having the following amino acid sequences:

Fragment 1: A A F L G D I A L D E E D L G
Fragment 2: A F Q V Q Q A A D L
Fragment 3: N Y Q D M V V E G
Fragment 4: S T P A Q D V S R
Fragment 5: N Q E A L R
Fragment 6: L S E P D P S H T L E E
Fragment 7: F D A Y Y
Fragment 8: L K P S N ? A T I Q S I V E

A less highly purified preparation of protein from bovine bone is prepared according to a purification scheme similar to that described in Example I. The purification basically varies from that previously described by omission of the DE-52 column, the CM cellulose column and the mono s column, as well as a reversal in the order of the hydroxylapatite and heparin sepharose columns. Briefly, the concentrated crude 4 M extract is brought to 85% final concentration of ethanol at 4 degrees. The mixture is then centrifuged, and the precipitate redissolved in 50 mM Tris, 0.15 M NaCl, 6.0 M urea. This material is then fractionated on Heparin Sepharose as described. The Heparin bound material is fractionated on hydroxyapatite as described. The active fractions are pooled, concentrated, and fractionated on a high resolution gel filtration (TSK 30000 in 6 M guanidinium chloride, 50 mM Tris, pH 7.2). The active fractions are pooled, dialyzed against 0.1% TFA, and then fractionated on a C4 Vydac reverse phase column as described. The preparation is reduced and electrophoresed on an acrylamide gel. The protein corresponding to the 18K band is eluted and digested with trypsin. Tryptic fragments are isolated having the following amino acid sequences:

Fragment 9: S L K P S N H A T I Q S ? V
Fragment 10: S F D A Y Y C S ? A
Fragment 11: V Y P N M T V E S C A
Fragment 12: V D F A D I ? W

Tryptic Fragments 7 and 8 are noted to be substantially the same as Fragments 10 and 9, respectively.

### A. bBMF-2

Two probes consisting of pools of oligonucleotides are designed on the basis of the amino acid sequence of Fragment 3 and synthesized on an automated DNA synthesizer as described above. 
Probe #1: A C N A C C A T [A/G] T C [T/C] T G [A/G] A T
Probe #2: C A [A/G] G A [T/C] A T G G T N G T N G A
 These probes are radioactively labeled and employed to screen the bovine genomic library constructed as follows: Bovine liver DNA is partially digested with the restriction endonuclease enzyme Sau 3A and sedimented through a sucrose gradient. Size fractionated DNA in the range of 15-30kb is then ligated to the lambda J1 BamH1 arms vector [Frischauf et al, J. Mol. Biol., 170:827-842 (1983) Mullins et al., Nature 308: 856-858 (1984)]. The library is plated at 8000 recombinants per plate. Duplicate nitrocellulose replicas of the plaques are made and amplified according to a modification of the procedure of Woo et al, Proc. Natl. Acad. Sci. USA, 75:3688-91 (1978).

The radioactively labelled 17-mer Probe #1 is hybridized to the set of filters according to the following method:

The probe is kinased and hybridized to the other set of filters in 3M tetramethylammonium chloride (TMAC) , 0.1M sodium phosphate pH6.5, 1mM EDTA, 5X Denhardts, 0.6% SDS, 100ug/ml salmon sperm DNA at 48 degrees C, and washed in 3M TMAC, 50mM Tris pH8.0 at 50 degrees C. These conditions minimize the detection of mismatches to the probe pool [see, Wood et al, Proc. Natl. Acad. Sci, U.S.A., 82:1585-1588 (1985)]. 400,000 recombinants are screened by this procedure. One duplicate positive is plaque purified and the DNA is isolated from a plate lysate of the recombinant bacteriophage designated lambda bP-21. Bacteriophage bP-21 was deposited with the ATCC under accession number ATCC 40310 on March 6, 1987. The bP-21 clone encodes the bovine growth factor designated bBMP-2.

The oligonucleotide hybridizing region of this bBMP-2 clone is localized to an approximately 1.2 kb Sac I restriction fragment which is subcloned into M13 and sequenced by standard techniques. The partial DNA sequence and derived amino acid sequence of this Sac I fragment and the contiguous Hind III-Sac I restriction fragment of bP-21 are shown below in Table II. The bBMP-2 peptide sequence from this clone is 129 amino acids in length and is encoded by the DNA sequence from nucleotide #1 through nucleotide #387. The amino acid sequence corresponding to the tryptic fragment isolated from the bovine bone 28 to 30kd material is underlined in Table II. The underlined portion of the sequence corresponds to tryptic Fragment 3 above from which the oligonucleotide probes for bBMP-2 are designed. The predicted amino acid sequence indicates that tryptic Fragment 3 is preceded by a basic residue (K) as expected considering the specificity of trypsin. The arginine residue encoded by the CGT triplet is presumed to be the carboxy-terminus of the protein based on the presence of a stop codon (TAG) adjacent to it.

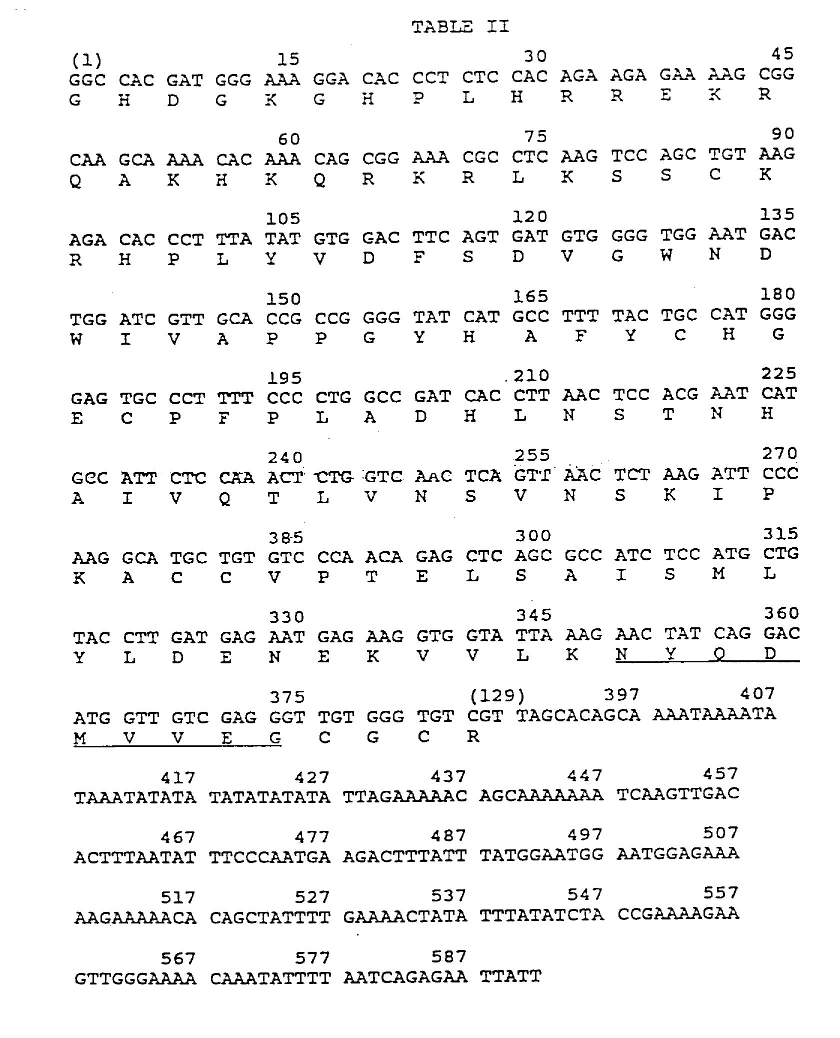

EXAMPLE V

### Human Bone Inductive Factors

### A. hBMP-2: Class I and II

The HindIII-SacI bovine genomic bBMP-2 fragment described in Example IV A. is subcloned into an M13 vector. A ³²p-labeled single-stranded DNA probe is made from a template preparation of this subclone. This probe is used to screen polyadenylated RNAs from various cell and tissue sources.

Polyadenylated RNAs from various cell and tissue sources are electrophoresed on formaldehyde-agarose gels and transferred to nitrocellulose by the method of Toole et al., supra. The probe is then hybridized to the nitrocellulose blot in 50% formamide, 5 X SSC, 0.1% SDS, 40 mM sodium phosphate pH6.5, 100 µg/ml denatured salmon sperm DNA, and 5 mM vanadyl ribonucleosides at 42° C overnight and washed at 65° C in 0.2 X SSC, 0.1% SDS. Following autoradiography, a hybridizing band corresponding to an mRNA species of approximately 3.8 kb is detected in the lane containing RNA from the human cell line U-2 OS. The HindIII-SacI fragment is labeled with ³²P by nick translation and used to screen the nitrocellulose filter replicas of a U-2 OS cDNA library by hybridization in standard hybridization buffer at 65° overnight followed by washing in 1 X SSC, 0.1% SDS at 65°.

This library was constructed by synthesizing cDNA from U-2 OS polyadenylated RNA and cloning into lambda gt10 by established techniques (Toole et al., supra). Twelve duplicate positive clones are picked and replated for secondaries. Duplicate nitrocellulose replicas are made of the secondary plates and both sets hybridized to the bovine genomic probe as the primary screening was performed. One set of filters is then washed in 1 X SSC, 0.1% SDS; the other in 0.1 X SSC, 0.1% SDS at 65°.

Two classes of hBMP-2 cDNA clones are evident based on strong (4 recombinants) or weak (7 recombinants) hybridization signals under the more stringent washing conditions (0.1 X SSC, 0.1% SDS). All 11 recombinant bacteriophages are plaque purified, small scale DNA preparations made from plate lysates of each, and the inserts subcloned into pSP65 and into M13 for sequence analysis. Sequence analysis of the strongly hybridizing clones designated hBHP-2 Class I (also known as BMP-2) indicates that they have extensive sequence homology with the sequence given in Table II. These clones are therefore cDNA encoding the human equivalent of the protein encoded by the bBMP-2 gene whose partial sequence is given in Table II. Sequence analysis of the weakly hybridizing recombinants designated hBMP-2 Class II (also known as BMP-4) indicates that they are also quite homologous with the sequence given in Table II at the 3' end of their coding regions, but less so in the more 5' regions. Thus they encode a human protein of similar, though not identical, structure to that above.

Full length hBMP-2 Class I cDNA clones are obtained in the following manner. The 1.5 kb insert of one of the Class II subclones (II-10-1) is isolated and radioactively labeled by nick-translation. One set of the nitrocellulose replicas of the U-2 OS cDNA library screened above (50 filters, corresponding to 1,000,000 recombinant bacteriophage) is rehybridized with this probe under stringent conditions (hybridization at 65° in standard hybridization buffer; washing at 65° in 0.2 X SSC, 0.1% SDS). All recombinants which hybridize to the bovine genomic probe which do not hybridize to the Class II probe are picked and plaque purified (10 recombinants). Plate stocks are made and small scale bacteriophage DNA preparations made. After subcloning into M13, sequence analysis indicates that 4 of these represent clones which overlap the original Class I clone. One of these, lambda U2OS-39, contains an approximately 1.5 kb insert and was deposited with the ATCC on June 16, 1987 under accession number 40345. The partial DNA sequence (compiled from lambda U2OS-39 and several other hBMP-2 Class I cDNA recombinants) and derived amino acid sequence are shown below in Table III. Lambda U2OS-39 is expected to contain all of the nucleotide sequence necessary to encode the entire human counterpart of the protein BMP-2 Class I encoded by the bovine gene segment whose partial sequence is presented in Table II. This human cDNA hBMP-2 Class I contains an open reading frame of 1188 bp, encoding a protein of 396 amino acids. This protein of 396 amino acids has a molecular weight of 45kd based on this amino acid sequence. It is contemplated that this sequence represents the primary translation product. The protein is preceded by a 5' untranslated region of 342 bp with stop codons in all frames. The 13 bp region preceding this 5' untranslated region represents a linker used in the cDNA cloning procedure.

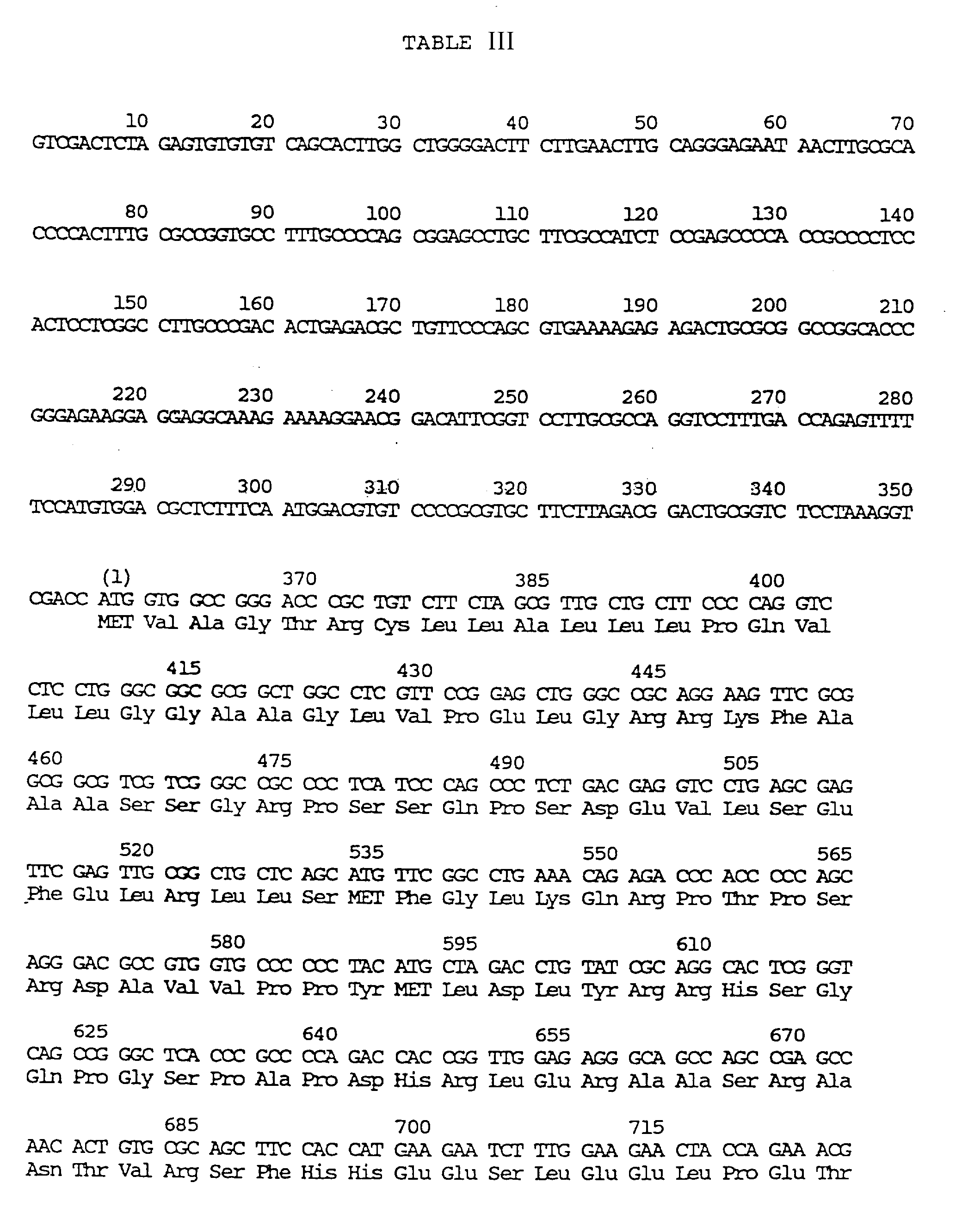

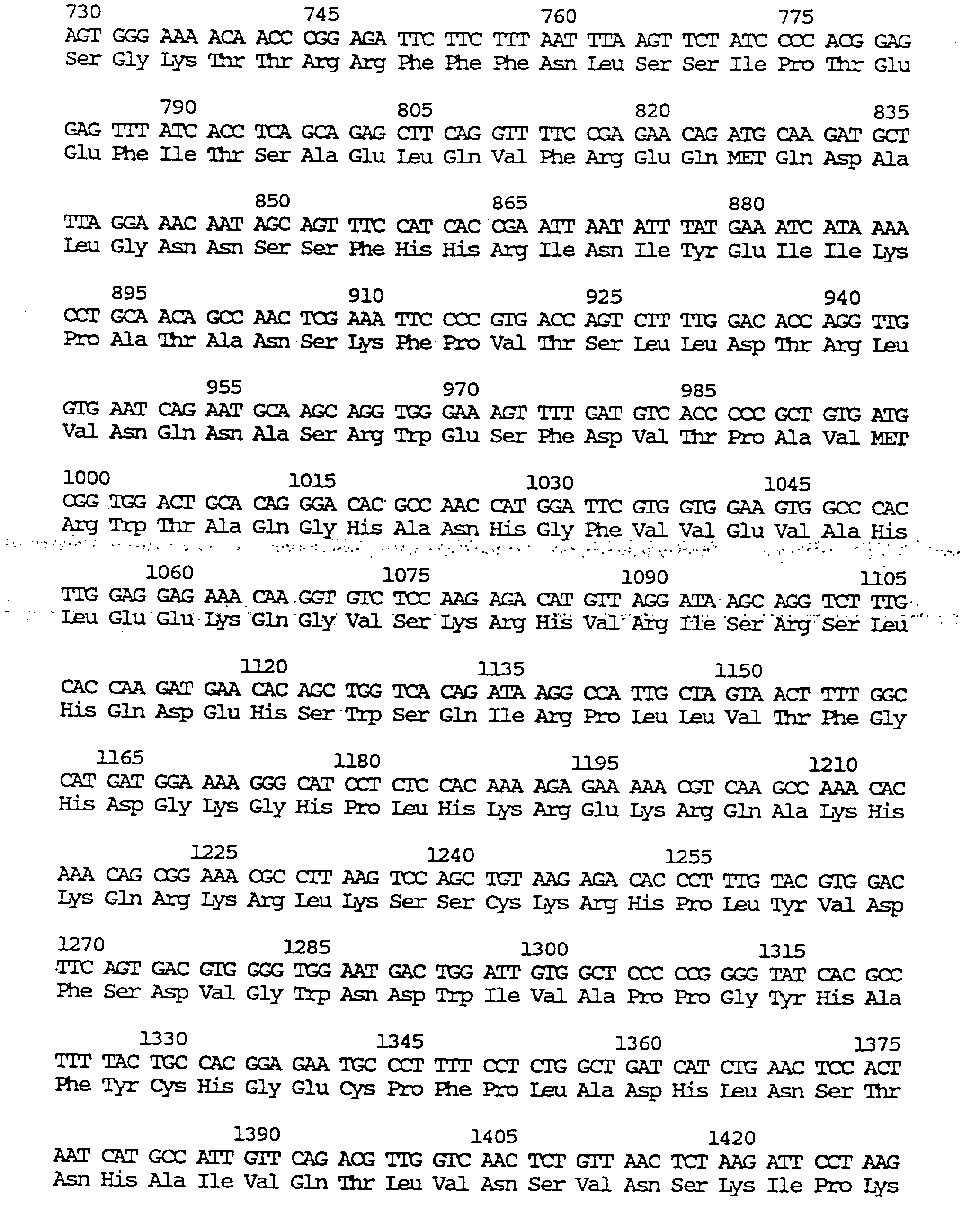

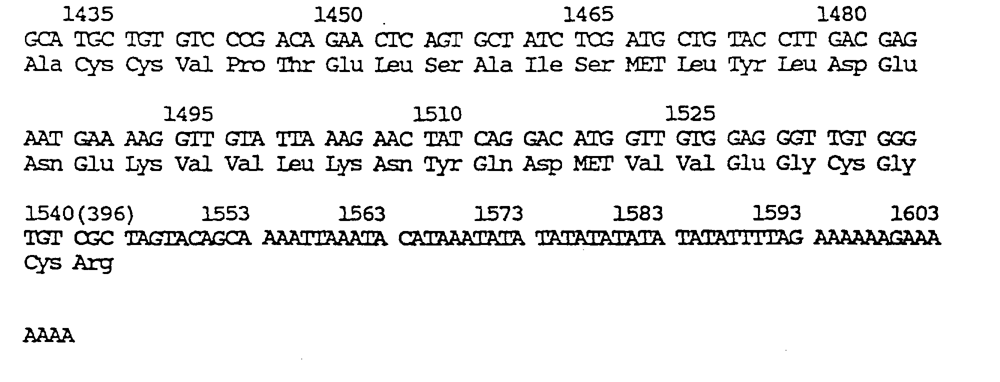

Full-length hBMP-2Class II human cDNA clones are obtained in the following manner. The 200 bp EcoRI-SacI fragment from the 5' end of the Class II recombinant II-10-1 is isolated from its plasmid subclone, labeled by nick-translation, and hybridized to a set of duplicate nitrocellulose replicas of the U-2 OS cDNA library (25 filters/set; representing 500,000 recombinants). Hybridization and washing are performed under stringent conditions as described above. 16 duplicate positives are picked and replated for secondaries. Nitrocellulose filter replicas of the secondary plates are made and hybridized to an oligonucleotide which was synthesized to correspond to the sequence of II-10-1 and is of the following sequence: CGGGCGCTCAGGATACTCAAGACCAGTGCTG Hybridization is in standard hybridization buffer at 50°C with washing at 50° in 1 X SSC, 0.1% SDS. 14 recombinant bacteriophages which hybridize to this oligonucleotide are plaque purified. Plate stocks are made and small scale bacteriophage DNA preparations made. After subcloning 3 of these into M13, sequence analysis indicates that they represent clones which overlap the original Class II clone. One of these, lambda U2OS-3, was deposited with the ATCC under accession number 40342 on June 16, 1987. U2OS-3 contains an insert of approximately 1.8 kb. The partial DNA sequence and derived amino acid sequence of U2OS-3 are shown below in Table IV. This clone is expected to contain all of the nucleotide sequence necessary to encode the entire human BMP-2 Class II protein. This cDNA contains an open reading frame of 1224 bp, encoding a protein of 408 amino acids, preceded by a 5' untranslated region of 394 bp with stop codons in all frames, and contains a 3' untranslated region of 308 bp following the in-frame stop codon. The 8 bp region preceding the 5' untranslated region represents a linker used in the cDNA cloning procedure. This protein of 408 amino acids has molecular weight of 47kd and is contemplated to represent the primary translation product.

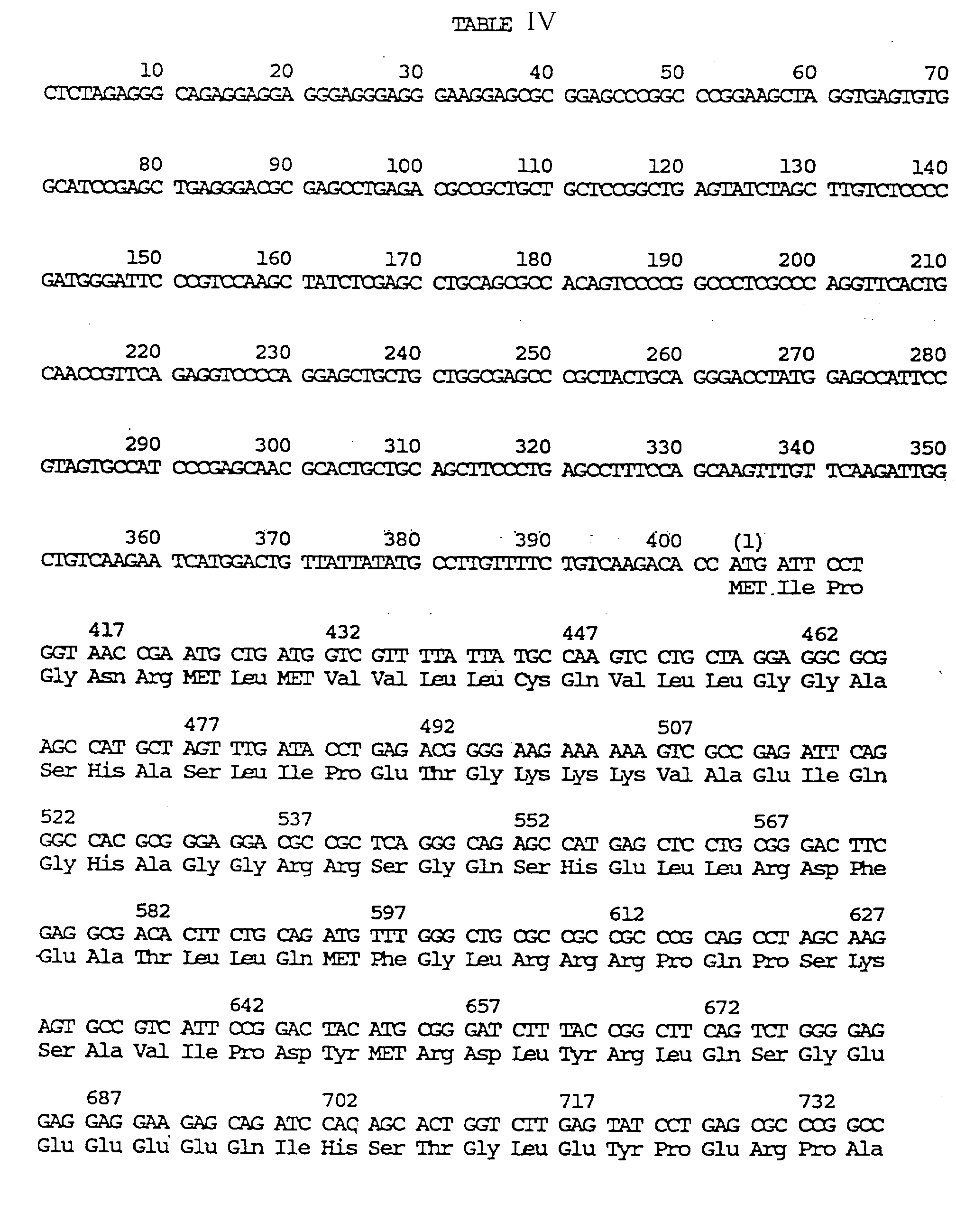

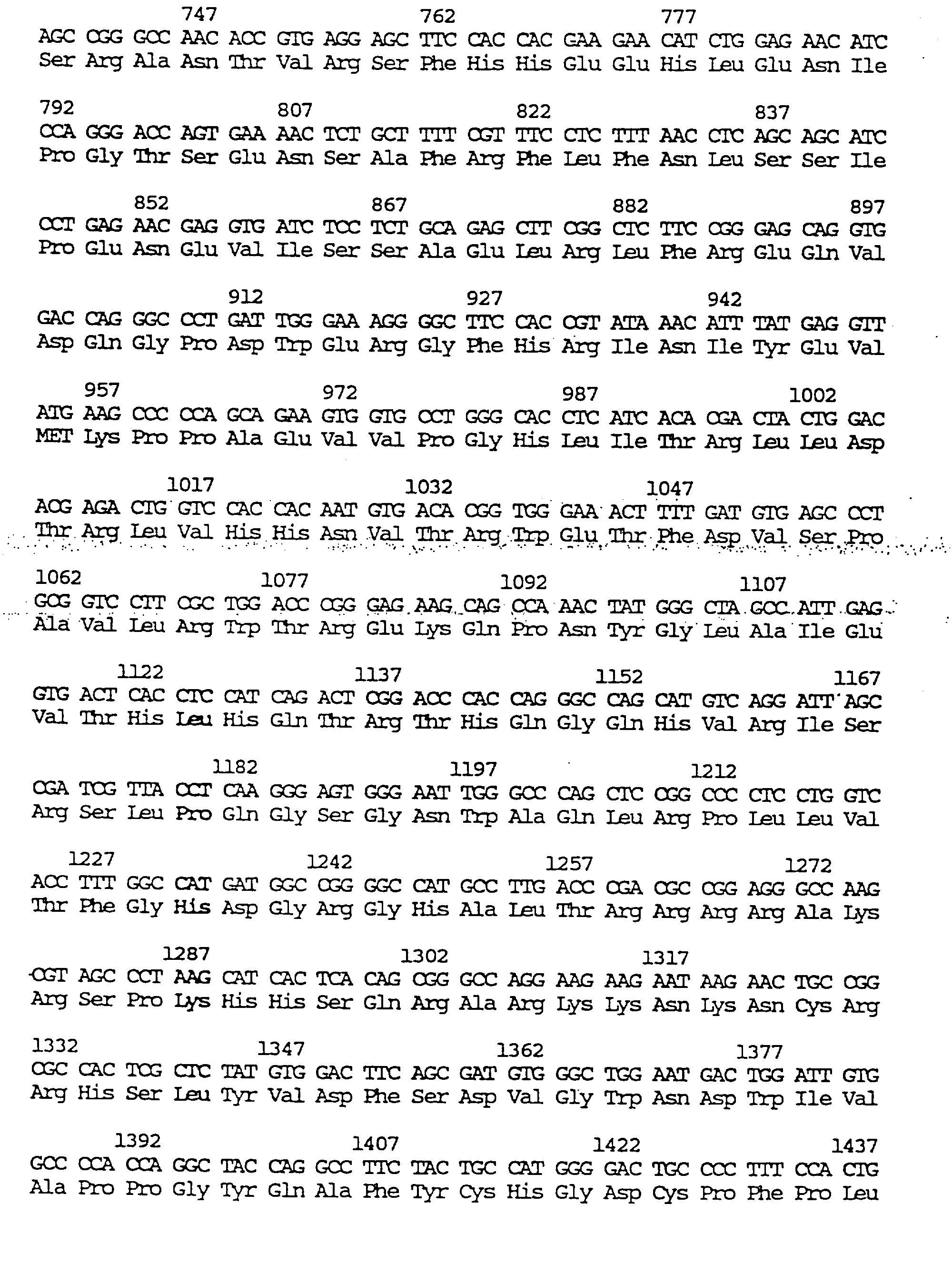

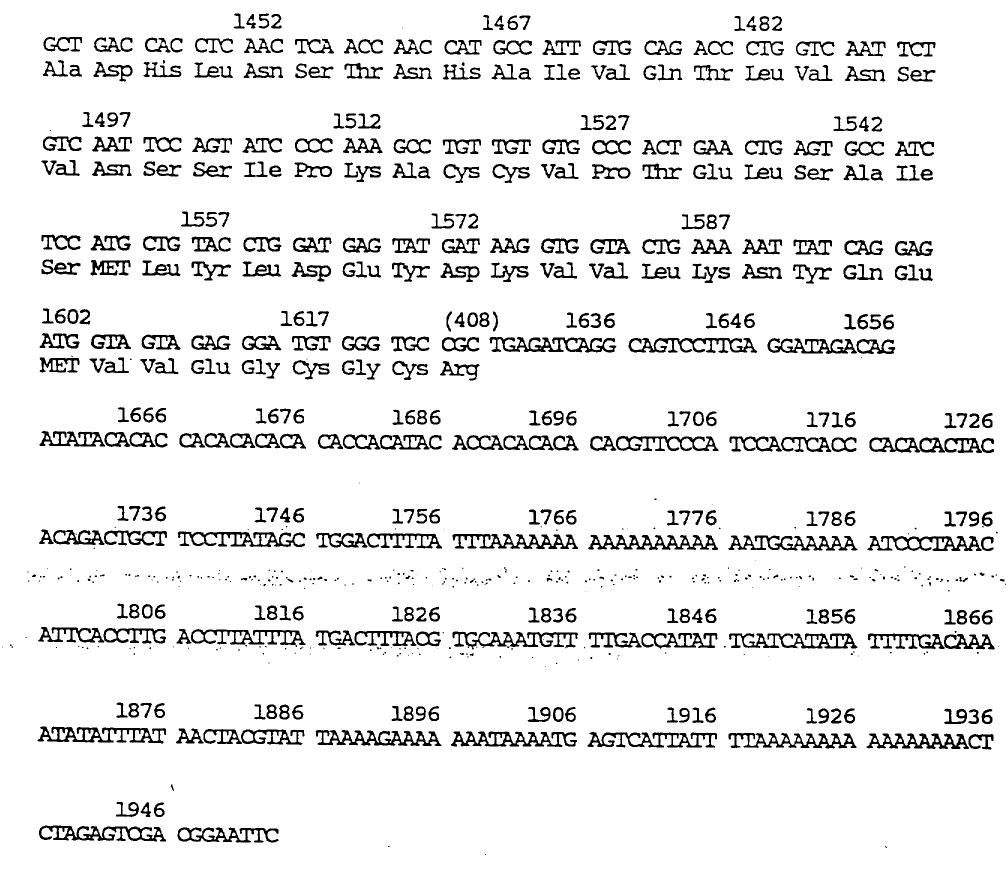

The sequences of BMP-2 Class I and II as shown in Tables II, III IV and have significant homology to the beta (B) and beta (A) subunits of the inhibins. The inhibins are a family of hormones which are presently being investigated for use in contraception. See, A. J. Mason et al, Nature, 318:659-663 (1985). To a lesser extent they are also homologous to Mullerian inhibiting substance (MIS), a testicular glycoprotein that causes regression of the Mullerian duct during development of the male embryo and transforming growth factor-beta (TGF-b) which can inhibit or stimulate growth of cells or cause them to differentiate. Furthermore, the sequence of Table IV encoding hBMP-2 Class II has significant homology to the Drosophila decapentaplegic (DPP-C) locus transcript. See, J. Massague, Cell, 49:437-438 (1987); R. W. Padgett et al, Nature, 325:81-84 (1987); R.L. Cate et al, Cell 45: 685-698 (1986). It is considered possible therefore that BMP-2 Class II is the human homolog of the protein made from this transcript form this developmental mutant locus.

### EXAMPLE VI

### Expression of Bone Inductive Factors.

In order to produce bovine, human or other mammalian bone inductive factors, the DNA encoding it is transferred into an appropriate expression vector and introduced into mammalian cells by conventional genetic engineering techniques.

One skilled in the art can construct mammalian expression vectors by employing the sequence of Tables II, III AND IV or other modified sequences and known vectors, such as pCD [Okayama et al., Mol. Cell Biol., 2:161-170 (1982)] and pJL3, pJL4 [Gough et al., EMBO J., 4:645-653 (1985)]. The transformation of these vectors into appropriate host cells can result in expression of osteoinductive factors. One skilled in the art could manipulate the sequences of Tables II, III and IV by eliminating or replacing the mammalian regulatory sequences flanking the coding sequence with bacterial sequences to create bacterial vectors for intracellular or extracellular expression by bacterial cells. For example, the coding sequences could be further manipulated (e.g. ligated to other known linkers or modified by deleting non-coding sequences there-from or altering nucleotides therein by other known techniques). The modified bone inductive factor coding sequence could then be inserted into a known bacterial vector using procedures such as described in T. Taniguchi et al., Proc. Natl Acad. Sci. USA, 77:5230-5233 (1980). This exemplary bacterial vector could then be transformed into bacterial host cells and bone inductive factor expressed thereby. For a strategy for producing extracellular expression of bone inductive factor in bacterial cells., see, e.g. European patent application EPA 177,343.

Similar manipulations can be performed for the construction of an insect vector [see, e.g. procedures described in published European patent application 155,476] for expression in insect cells. A yeast vector could also be constructed employing yeast regulatory sequences for intracellular or extracellular expression of the factors of the present invention by yeast cells. [See, e.g., procedures described in published PCT application W086/00639 and European patent application EPA 123,289].

A method for producing high levels of an osteoinductive factor of the invention from mammalian cells involves the construction of cells containing multiple copies of the heterologous bone inductive factor gene. The heterologous gene can be linked to an amplifiable marker, e.g. the dihydrofolate reductase (DHFR) gene for which cells containing increased gene copies can be selected for propagation in increasing concentrations of methotrexate (MTX) according to the procedures of Kaufman and Sharp, J. Mol. Biol., 159:601-629 (1982). This approach can be employed with a number of different cell types.

For example, a plasmid containing a DNA sequence for a bone inductive factor of the invention in operative association with other plasmid sequences enabling expression thereof and the DHFR expression plasmid pAdA26SV(A)3 [Kaufman and Sharp, Mol. Cell. Biol., 2:1304 (1982)] can be co-introduced into DHFR-deficient CHO cells, DUKX-BII, by calcium phosphate coprecipitation and transfection. DHFR expressing transformants are selected for growth in alpha media with dialyzed fetal calf serum, and subsequently selected for amplification by growth in increasing concentrations of MTX (sequential steps in 0.02, 0.2, 1.0 and 5uM MTX) as described in Kaufman et al., Mol Cell Biol., 5:1750 (1983). Transformants are cloned, and biologically active bone inductive factor expression is monitored by rat bone formation assay. Bone inductive factor expression should increase with increasing levels of MTX resistance. Similar procedures can be followed to produce other bone inductive factors.

Alternatively, the human gene is expressed directly, as described above. Active bone inductive factor may be produced in bacteria or yeast cells. However the presently preferred expression system for biologically active recombinant human bone inductive factor is stably transformed CHO cells.

As one specific example, to produce the human bone inductive factor (hBMP-1) of Example V, the insert of U2OS-1 is released from the vector arms by digestion with Sal I and subcloned into the mammalian expression vector pMT2CX digested with Xho I. Plasmid DNA from this subclone is transfected into COS cells by the DEAE-dextran procedure [Sompayrac and Danna PNAS 78:7575-7578 (1981); Luthman and Magnusson, Nucl.Acids Res. 11: 1295-1308 (1983)]. Serum-free 24 hr. conditioned medium is collected from the cells starting 40-70 hr. post-transfection.

The mammalian expression vector pMT2 Cla-Xho (pMT2 CX) is a derivative of p91023 (b) (Wong et al., Science 228:810-815, 1985) differing from the latter in that it contains the ampicillin resistance gene in place of the tetracycline resistance gene and further contains a XhoI site for insertion of cDNA clones. The functional elements of pMT2 Cla-Xho have been described (Kaufman, R.J., 1985, Proc. Natl. Acad. Sci. USA 82:689-693) and include the adenovirus VA genes, the SV40 origin of replication including the 72 bp enhancer, the adenovirus major late promoter including a 5' splice site and the majority of the adenovirus tripartite leader sequence present on adenovirus late mRNAs, a 3' splice acceptor site, a DHFR insert, the SV40 early polyadenylation site (SV40), and pBR322 sequences needed for propagation in E. coli.

Plasmid pMT2 Cla-Xho is obtained by EcoRI digestion of pMT2-VWF, which has been deposited with the American Type Culture Collection (ATCC), Rockville, MD (USA) under accession number ATCC 67122. EcoRI digestion excises the cDNA insert present in pMT2-VWF, yielding pMT2 in linear form which can be ligated and used to transform E. coli HB 101 or DH-5 to ampicillin resistance. Plasmid pMT2 DNA can be prepared by conventional methods. pMT2CX is then constructed by digesting pMT2 with Eco RV and XbaI, treating the digested DNA with Klenow fragment of DNA polymerase I, and ligating Cla linkers (NEBiolabs, CATCGATG). This removes bases 2266 to 2421 starting from the Hind III site near the SV40 origin of replication and enhancer sequences of pMT2. Plasmid DNA is then digested with EcoRI, blunted as above, and ligated to an EcoRI adapter, 5' PO4-AATTCCTCGAGAGCT 3' 3' GGAGCTCTCGA 5' digested with XhoI, and ligated, yielding pMT2 Cla-Xho, which may then be used to transform E. coli to ampicillin resistance. Plasmid pMT2 Cla-Xho DNA may be prepared by conventional methods.

### Example VII

### Biological Activity of Expressed Bone Inductive Factor

### A. BMP-1

To measure the biological activity of the expressed bone inductive factor. (hBMP-1) obtained in Example VI above. The factor is partially purified on a Heparin Sepharose column. 4 ml of transfection supernatant from one 100 mm dish is concentrated approximately 10 fold by ultrafiltration on a YM 10 membrane and then dialyzed against 20mM Tris, 0.15 M NaCl, pH 7.4 (starting buffer). This material is then applied to a 1.1 ml Heparin Sepharose column in starting buffer. Unbound proteins are removed by an 8 ml wash of starting buffer, and bound proteins, including BMP-1, are desorbed by a 3-4 ml wash of 20 mM Tris, 2.0 M NaCl, pH 7.4.

The proteins bound by the Heparin column are concentrated approximately 10-fold on a Centricon 10 and the salt reduced by diafiltration with 0.1% trifluoroacetic acid. The appropriate amount of this solution is mixed with 20 mg of rat matrix and then assayed for in vivo bone and cartilage formation as previously described in Example III. A mock transfection supernatant fractionation is used as a control.

The implants containing rat matrix to which specific amounts of human BMP-1 have been added are removed from rats after seven days and processed for histological evaluation. Representative sections from each implant are stained for the presence of new bone mineral with von Kossa and acid fuschin, and for the presence of cartilage-specific matrix formation using toluidine blue. The types of cells present within the section, as well as the extent to which these cells display phenotype are evaluated.

Addition of human BMP-1 to the matrix material resulted in formation of cartilage-like nodules at 7 days post implantation. The chondroblast-type cells were recognizable by shape and expression of metachromatic matrix. The amount of activity observed for human BMP-1 was dependent upon the amount of human BMP-1 protein added to the matrix. Table IX illustrates the dose-response relationship of human BMP-1 protein to the amount of bone induction observed.

**Table IX**

| IMPLANT NUMBER | AMOUNT USED (equivalent of ml transfection media) | HISTOLOGICAL SCORE |
|---|---|---|
| 876-134-1 | 10 BMP-1 | C+2 |
| 876-134-2 | 3 BMP-1 | C+1 |
| 876-134-3 | 1 BMP-1 | C+/- |
| 876-134-4 | 10 MOCK | C- |
| 876-134-5 | 3 MOCK | C - |
| 876-134-6 | 1 MOCK | C - |

Cartilage (c) activity was scored on a scale from 0(-) to 5.

Similar levels of activity are seen in the Heparin Sepharose fractionated COS cell extracts. Partial purification is accomplished in a similar manner as described above except that 6 M urea is included in all the buffers. Further, in a rat bone formation assay as described above, BMP-2 has similarly demonstrated chondrogenic activity.

The procedures described above may be employed to isolate other bone inductive factors of interest by utilizing the bovine bone inductive factors and/or human bone inductive factors as a probe source. Such other bone inductive factors may find similar utility in, inter alia, fracture repair.

The foregoing descriptions detail presently preferred embodiments of the present invention. Numerous modifications and variations in practice thereof are expected to occur to those skilled in the art upon consideration of these descriptions. Those modifications and variations are believed to be encompassed within the claims appended hereto.

## Claims (Claims for the following Contracting State(s): BE, CH, DE, FR, GB, IT, LI, LU, NL, SE)

1. A gene encoding human BMP-2 comprising the following DNA sequence:

10 20 30 40 50 60 70
GTCGACTCTA GAGTGTGTGT CAGCACTTGG CTGGGGACTT CTTGAACTTG CAGGGAGAAT AACTTGCGCA

80 90 100 110 120 130 140
CCCCACTTTG CGCCGGTGCC TTTGCCCCAG CGGAGCCTGC TTCGCCATCT CCGAGCCCCA CCGCCCCTCC

150 160 170 180 190 200 210
ACTCCTCGGC CTTGCCCGAC ACTGAGACGC TGTTCCCAGC GTGAAAAGAG AGACTGCGCG GCCGGCACCC

220 230 240 250 260 270 280
GGGAGAAGGA GGAGGCAAAG AAAAGGAACG GACATTCGGT CCTTGCGCCA GGTCCTTTGA CCAGAGTTTT

290 300 310 320 330 340 350
TCCATGTGGA CGCTCTTTCA ATGGACGTGT CCCCGCGTGC TTCTTAGACG GACTGCGGTC TCCTAAAGGT

(1) 370 385 400
CGACC ATG GTG GCC GGG ACC CGC TGT CTT CTA GCG TTG CTG CTT CCC CAG GTC


415 430 445
CTC CTG GGC GGC GCG GCT GGC CTC GTT CCG GAG CTG GGC CGC AGG AAG TTC GCG


460 475 490 505
GCG GCG TCG TCG GGC CGC CCC TCA TCC CAG CCC TCT GAC GAG GTC CTG AGC GAG


520 535 550 565
TTC GAG TTG CGG CTG CTC AGC ATG TTC GGC CTG AAA CAG AGA CCC ACC CCC AGC


580 595 610
AGG GAC GCC GTG GTG CCC CCC TAC ATG CTA GAC CTG TAT CGC AGG CAC TCG GGT


625 640 655 670
CAG CCG GGC TCA CCC GCC CCA GAC CAC CGG TTG GAG AGG GCA GCC AGC CGA GCC


685 700 715
AAC ACT GTG CGC AGC TTC CAC CAT GAA GAA TCT TTG GAA GAA CTA CCA GAA ACG


730 745 760 775
AGT GGG AAA ACA ACC CGG AGA TTC TTC TTT AAT TTA AGT TCT ATC CCC ACG GAG


790 805 820 835
GAG TTT ATC ACC TCA GCA GAG CTT CAG GTT TTC CGA GAA CAG ATG CAA GAT GCT


850 865 880
TTA GGA AAC AAT AGC AGT TTC CAT CAC CGA ATT AAT ATT TAT GAA ATC ATA AAA


895 910 925 940
CCT GCA ACA GCC AAC TCG AAA TTC CCC GTG ACC AGT CTT TTG GAC ACC AGG TTG


955 970 985
GTG AAT CAG AAT GCA AGC AGG TGG GAA AGT TTT GAT GTC ACC CCC GCT GTG ATG


1000 1015 1030 1045
CGG TGG ACT GCA CAG GGA CAC GCC AAC CAT GGA TTC GTG GTG GAA GTG GCC CAC


1060 1075 1090 1105
TTG GAG GAG AAA CAA GGT GTC TCC AAG AGA CAT GTT AGG ATA AGC AGG TCT TTG


1120 1135 1150
CAC CAA GAT GAA CAC AGC TGG TCA CAG ATA AGG CCA TTG CTA GTA ACT TTT GGC


1165 1180 1195 1210
CAT GAT GGA AAA GGG CAT CCT CTC CAC AAA AGA GAA AAA CGT CAA GCC AAA CAC


1225 1240 1255
AAA CAG CGG AAA CGC CTT AAG TCC AGC TGT AAG AGA CAC CCT TTG TAC GTG GAC


1270 1285 1300 1315
TTC AGT GAC GTG GGG TGG AAT GAC TGG ATT GTG GCT CCC CCG GGG TAT CAC GCC


1330 1345 1360 1375
TTT TAC TGC CAC GGA GAA TGC CCT TTT CCT CTG GCT GAT CAT CTG AAC TCC ACT


1390 1405 1420
AAT CAT GCC ATT GTT CAG ACG TTG GTC AAC TCT GTT AAC TCT AAG ATT CCT AAG


1435 1450 1465 1480
GCA TGC TGT GTC CCG ACA GAA CTC AGT GCT ATC TCG ATG CTG TAC CTT GAC GAG


1495 1510 1525
AAT GAA AAG GTT GTA TTA AAG AAC TAT CAG GAC ATG GTT GTG GAG GGT TGT GGG


1540(396) 1553 1563 1573 1583 1593 1603
TGT CGC TAGTACAGCA AAATTAAATA CATAAATATA TATATATATA TATATTTTAG AAAAAAGAAA


AAAA

2. A gene encoding human BMP-2 having the amino acid sequence given in claim 1.

3. A gene encoding a protein exhibiting properties of human BMP-2 and comprising a DNA sequence:
(a) which differs from a DNA sequence of claim 1 in codon sequence due to the degeneracy of the genetic code;
(b) which hybridises with a DNA sequence of claim 1 or section (a), above; or
(c) represents a fragment, allelic or other variation of a DNA sequence of claim 1, whether said variation results in changes in the peptide sequence or not.

4. The DNA sequence of claim 3, which is a genomic DNA sequence.

5. The DNA sequence of claim 3, which is a cDNA sequence.

6. A gene encoding bovine BMP-2 comprising the following DNA sequence:

(1)                    15                      30                      45
GGC CAC GAT GGG AAA GGA CAC CCT CTC CAC AGA AGA GAA AAG CGG
G   H   D   G   K   G   H   P   L   H   R   R   E   K   R

                          60                      75                      90
CAA GCA AAA CAC AAA CAG CGG AAA CGC CTC AAG TCC AGC TGT AAG
Q   A   K   H   K   Q   R   K   R   L   K   S   S   C   K

                          105                     120                     135
AGA CAC CCT TTA TAT GTG GAC TTC AGT GAT GTG GGG TGG AAT GAC
R   H   P   L   Y   V   D   F   S   D   V   G   W   N   D

                          150                     165                     180
TGG ATC GTT GCA CCG CCG GGG TAT CAT GCC TTT TAC TGC CAT GGG
W   I   V   A   P   P   G   Y   H   A   F   Y   C   H   G

                          195                     210                     225
GAG TGC CCT TTT CCC CTG GCC GAT CAC CTT AAC TCC ACG AAT CAT
E   C   P   F   P   L   A   D   H   L   N   S   T   N   H

                          240                     255                     270
GCC ATT CTC CAA ACT CTG GTC AAC TCA GTT AAC TCT AAG ATT CCC
A   I   V   Q   T   L   V   N   S   V   N   S   K   I   P

                          385                     300                     315
AAG GCA TGC TGT GTC CCA ACA GAG CTC AGC GCC ATC TCC ATG CTG
K   A   C   C   V   P   T   E   L   S   A   I   S   M   L

                          330                     345                     360
TAC CTT GAT GAG AAT GAG AAG GTG GTA TTA AAG AAC TAT CAG GAC
Y   L   D   E   N   E   K   V   V   L   K   N   Y   Q   D

                          375                     (129)       397         407
ATG GTT GTC GAG GGT TGT GGG TGT CGT TAGCACAGCA AAATAAAATA
M   V   V   E   G   C   G   C   R

        417        427        437        447        457
TAAATATATA TATATATATA TTAGAAAAAC AGCAAAAAAA TCAAGTTGAC

        467        477        487        497        507
ACTTTAATAT TTCCCAATGA AGACTTTATT TATGGAATGG AATGGAGAAA

        517        527        537        547        557
AAGAAAAACA CAGCTATTTT GAAAACTATA TTTATATCTA CCGAAAAGAA

        567        577        587
GTTGGGAAAA CAAATATTTT AATCAGAGAA TTATT

7. A gene encoding bovine BMP-2 containing the amino acid sequence of claim 6.

8. A gene encoding a protein exhibiting properties of bovine BMP-2 and comprising DNA sequences:
(a) which differ from a DNA sequence of claim 7 in codon sequence due to the degeneracy of the genetic code;
(b) which hybridise with a DNA sequence of claim 7 or section (a), above; or
(c) represent fragments, allelic or other variations of a DNA sequence of claim 7, whether said variations result in changes in the peptide sequence or not.

9. The DNA sequence of claim 8, which is a genomic DNA sequence.

10. The DNA sequence of claim 8, which is a cDNA sequence.

11. A gene encoding human BMP-4 comprising the following DNA sequence:

10 20 30 40 50 60 70
CTCTAGAGGG CAGAGGAGGA GGGAGGGAGG GAAGGAGCGC GGAGCCCGGC CCGGAAGCTA GGTGAGTGTG

80 90 100 110 120 130 140
GCATCCGAGC TGAGGGACGC GAGCCTGAGA CGCCGCTGCT GCTCCGGCTG AGTATCTAGC TTGTCTCCCC

150 160 170 180 190 200 210
GATGGGATTC CCGTCCAAGC TATCTCGAGC CTGCAGCGCC ACAGTCCCCG GCCCTCGCCC AGGTTCACTG

220 230 240 250 260 270 280
CAACCGTTCA GAGGTCCCCA GGAGCTGCTG CTGGCGAGCC CGCTACTGCA GGGACCTATG GAGCCATTCC

290 300 310 320 330 340 350
GTAGTGCCAT CCCGAGCAAC GCACTGCTGC AGCTTCCCTG AGCCTTTCCA GCAAGTTTGT TCAAGATTGG

360 370 380 390 400 (1)
CTGTCAAGAA TCATGGACTG TTATTATATG CCTTGTTTTC TGTCAAGACA CC ATG ATT CCT


417 432 447 462
GGT AAC CGA ATG CTG ATG GTC GTT TTA TTA TGC CAA GTC CTG CTA GGA GGC GCG


477 492 507
AGC CAT GCT AGT TTG ATA CCT GAG ACG GGG AAG AAA AAA GTC GCC GAG ATT CAG


522 537 552 567
GGC CAC GCG GGA GGA CGC CGC TCA GGG CAG AGC CAT GAG CTC CTG CGG GAC TTC


582 597 612 627
GAG GCG ACA CTT CTG CAG ATG TTT GGG CTG CGC CGC CGC CCG CAG CCT AGC AAG


642 657 672
AGT GCC GTC ATT CCG GAC TAC ATG CGG GAT CTT TAC CGG CTT CAG TCT GGG GAG


687 702 717 732
GAG GAG GAA GAG CAG ATC CAC AGC ACT GGT CTT GAG TAT CCT GAG CGC CCG GCC


747 762 777
AGC CGG GCC AAC ACC GTG AGG AGC TTC CAC CAC GAA GAA CAT CTG GAG AAC ATC


792 807 822 837
CCA GGG ACC AGT GAA AAC TCT GCT TTT CGT TTC CTC TTT AAC CTC AGC AGC ATC


852 867 882 897
CCT GAG AAC GAG GTG ATC TCC TCT GCA GAG CTT CGG CTC TTC CGG GAG CAG GTG


912 927 942
GAC CAG GGC CCT GAT TGG GAA AGG GGC TTC CAC CGT ATA AAC ATT TAT GAG GTT


957 972 987 1002
ATG AAG CCC CCA GCA GAA GTG GTG CCT GGG CAC CTC ATC ACA CGA CTA CTG GAC


1017 1032 1047
ACG AGA CTG GTC CAC CAC AAT GTG ACA CGG TGG GAA ACT TTT GAT GTG AGC CCT


1062 1077 1092 1107
GCG GTC CTT CGC TGG ACC CGG GAG AAG CAG CCA AAC TAT GGG CTA GCC ATT GAG


1122 1137 1152 1167
GTG ACT CAC CTC CAT CAG ACT CGG ACC CAC CAG GGC CAG CAT GTC AGG ATT AGC


1182 1197 1212
CGA TCG TTA CCT CAA GGG AGT GGG AAT TGG GCC CAG CTC CGG CCC CTC CTG GTC


1227 1242 1257 1272
ACC TTT GGC CAT GAT GGC CGG GGC CAT GCC TTG ACC CGA CGC CGG AGG GCC AAG


1287 1302 1317
CGT AGC CCT AAG CAT CAC TCA CAG CGG GCC AGG AAG AAG AAT AAG AAC TGC CGG


1332 1347 1362 1377
CGC CAC TCG CTC TAT GTG GAC TTC AGC GAT GTG GGC TGG AAT GAC TGG ATT GTG


1392 1407 1422 1437
GCC CCA CCA GGC TAC CAG GCC TTC TAC TGC CAT GGG GAC TGC CCC TTT CCA CTG


1452 1467 1482
GCT GAC CAC CTC AAC TCA ACC AAC CAT GCC ATT GTG CAG ACC CTG GTC AAT TCT


1497 1512 1527 1542
GTC AAT TCC AGT ATC CCC AAA GCC TGT TGT GTG CCC ACT GAA CTG AGT GCC ATC


1557 1572 1587
TCC ATG CTG TAC CTG GAT GAG TAT GAT AAG GTG GTA CTG AAA AAT TAT CAG GAG


1602 1617 (408) 1636 1646 1656
ATG GTA GTA GAG GGA TGT GGG TGC CGC TGAGATCAGG CAGTCCTTGA GGATAGACAG


1666 1676 1686 1696 1706 1716 1726
ATATACACAC CACACACACA CACCACATAC ACCACACACA CACGTTCCCA TCCACTCACC CACACACTAC

1736 1746 1756 1766 1776 1786 1796
ACAGACTGCT TCCTTATAGC TGGACTTTTA TTTAAAAAAA AAAAAAAAAA AATGGAAAAA ATCCCTAAAC

1806 1816 1826 1836 1846 1856 1866
ATTCACCTTG ACCTTATTTA TGACTTTACG TGCAAATGTT TTGACCATAT TGATCATATA TTTTGACAAA

1876 1886 1896 1906 1916 1926 1936
ATATATTTAT AACTACGTAT TAAAAGAAAA AAATAAAATG AGTCATTATT TTAAAAAAAA AAAAAAAACT

1946
CTAGAGTCGA CGGAATTC

12. A gene encoding human BMP-4 having the amino acid sequence given in claim 11.

13. A gene encoding a protein exhibiting properties of BMP-4 and comprising a DNA sequence:
(a) which differs from a DNA sequence of claim 11 in codon sequence due to the degeneracy of the genetic code;
(b) which hybridises with a DNA sequence of claim 11 or section (a), above; or
(c) represents a fragment, allelic or other variation of a DNA sequence of claim 11, whether said variation results in changes in the peptide sequence or not.

14. The DNA sequence of claim 13, which is a genomic DNA sequence.

15. The DNA sequence of claim 13, which is a cDNA sequence.

16. A vector containing the gene or DNA sequence of any one of claims 1 to 15 in operative association with an expression control sequence.

17. A cell transformed with a vector of claim 16.

18. The cell of claim 17 which is a mammalian cell, a bacterial cell, an insect cell or a yeast cell.

19. The cell of claim 18 which is a CHO cell.

20. A protein exhibiting properties of BMP-2 which is encoded by a gene or DNA sequence of any one of claims 1 to 10.

21. A protein exhibiting properties of BMP-2, which is obtainable by the steps of culturing in a suitable culture medium a cell transformed with an expression vector comprising a gene or a DNA sequence of any one of claims 1 to 10, and recovering said protein from said culture medium.

22. A protein exhibiting properties of BMP-4 which is encoded by a gene or DNA sequence of any one of claims 11 to 15.

23. A protein exhibiting properties of BMP-4, which is obtainable by the steps of culturing in a suitable culture medium a cell transformed with an expression vector comprising a gene or a DNA sequence of any one of claims 11 to 15, and recovering said protein from said culture medium.

24. A process for producing the protein of claims 21 or 23, comprising the steps of culturing in a suitable culture medium the cell of claim 17 and isolating said protein from said culture medium.

25. A pharmaceutical composition comprising the proteins of any one of claims 20 to 23, individually or in combination, and a pharmaceutically acceptable vehicle.

26. The pharmaceutical composition of claim 25, further comprising a matrix capable of delivering the composition to the site of the bone or cartilage defect and providing a structure for inducing bone or cartilage formation.

27. The pharmaceutical composition of claim 26, wherein said matrix comprises hydroxyapatite, collagen, polylactic acid or tricalcium phosphate.

28. Use of a protein of any one of claims 20 to 23, individually or in combination, for the preparation of a pharmaceutical composition for inducing bone or cartilage formation.

## Claims (Claims for the following Contracting State(s): AT)

1. A process for the preparation of a gene encoding human BMP-2 comprising the following DNA sequence: 
10 20 30 40 50 60 70
GTCGACTCTA GAGTGTGTGT CAGCACTTGG CTGGGGACTT CTTGAACTTG CAGGGAGAAT AACTTGCGCA

80 90 100 110 120 130 140
CCCCACTTTG CGCCGGTGCC TTTGCCCCAG CGGAGCCTGC TTCGCCATCT CCGAGCCCCA CCGCCCCTCC

150 160 170 180 190 200 210
ACTCCTCGGC CTTGCCCGAC ACTGAGACGC TGTTCCCAGC GTGAAAAGAG AGACTGCGCG GCCGGCACCC

220 230 240 250 260 270 280
GGGAGAAGGA GGAGGCAAAG AAAAGGAACG GACATTCGGT CCTTGCGCCA GGTCCTTTGA CCAGAGTTTT

290 300 310 320 330 340 350
TCCATGTGGA CGCTCTTTCA ATGGACGTGT CCCCGCGTGC TTCTTAGACG GACTGCGGTC TCCTAAAGGT

(1) 370 385 400
CGACC ATG GTG GCC GGG ACC CGC TGT CTT CTA GCG TTG CTG CTT CCC CAG GTC


415 430 445
CTC CTG GGC GGC GCG GCT GGC CTC GTT CCG GAG CTG GGC CGC AGG AAG TTC GCG


460 475 490 505
GCG GCG TCG TCG GGC CGC CCC TCA TCC CAG CCC TCT GAC GAG GTC CTG AGC GAG


520 535 550 565
TTC GAG TTG CGG CTG CTC AGC ATG TTC GGC CTG AAA CAG AGA CCC ACC CCC AGC


580 595 610
AGG GAC GCC GTG GTG CCC CCC TAC ATG CTA GAC CTG TAT CGC AGG CAC TCG GGT


625 640 655 670
CAG CCG GGC TCA CCC GCC CCA GAC CAC CGG TTG GAG AGG GCA GCC AGC CGA GCC


685 700 715
AAC ACT GTG CGC AGC TTC CAC CAT GAA GAA TCT TTG GAA GAA CTA CCA GAA ACG

 
730 745 760 775
AGT GGG AAA ACA ACC CGG AGA TTC TTC TTT AAT TTA AGT TCT ATC CCC ACG GAG


790 805 820 835
GAG TTT ATC ACC TCA GCA GAG CTT CAG GTT TTC CGA GAA CAG ATG CAA GAT GCT


850 865 880
TTA GGA AAC AAT AGC AGT TTC CAT CAC CGA ATT AAT ATT TAT GAA ATC ATA AAA


895 910 925 940
CCT GCA ACA GCC AAC TCG AAA TTC CCC GTG ACC AGT CTT TTG GAC ACC AGG TTG


955 970 985
GTG AAT CAG AAT GCA AGC AGG TGG GAA AGT TTT GAT GTC ACC CCC GCT GTG ATG


1000 1015 1030 1045
CGG TGG ACT GCA CAG GGA CAC GCC AAC CAT GGA TTC GTG GTG GAA GTG GCC CAC


1060 1075 1090 1105
TTG GAG GAG AAA CAA GGT GTC TCC AAG AGA CAT GTT AGG ATA AGC AGG TCT TTG


1120 1135 1150
CAC CAA GAT GAA CAC AGC TGG TCA CAG ATA AGG CCA TTG CTA GTA ACT TTT GGC


1165 1180 1195 1210
CAT GAT GGA AAA GGG CAT CCT CTC CAC AAA AGA GAA AAA CGT CAA GCC AAA CAC


1225 1240 1255
AAA CAG CGG AAA CGC CTT AAG TCC AGC TGT AAG AGA CAC CCT TTG TAC GTG GAC


1270 1285 1300 1315
TTC AGT GAC GTG GGG TGG AAT GAC TGG ATT GTG GCT CCC CCG GGG TAT CAC GCC


1330 1345 1360 1375
TTT TAC TGC CAC GGA GAA TGC CCT TTT CCT CTG GCT GAT CAT CTG AAC TCC ACT


1390 1405 1420
AAT CAT GCC ATT GTT CAG ACG TTG GTC AAC TCT GTT AAC TCT AAG ATT CCT AAG

 
1435 1450 . 1465 1480
GCA TGC TGT GTC CCG ACA GAA CTC AGT GCT ATC TCG ATG CTG TAC CTT GAC GAG


1495 1510 1525
AAT GAA AAG GTT GTA TTA AAG AAC TAT CAG GAC ATG GTT GTG GAG GGT TGT GGG


1540(396) 1553 1563 1573 1583 1593 1603
TGT CGC TAGTACAGCA AAATTAAATA CATAAATATA TATATATATA TATATTTTAG AAAAAGAAA


AAAA ,
 wherein said process comprises the following steps:
a) screening of a gene library constructed from U-2 OS derived DNA or cDNA with a labelled bBMP-2 fragment by hybridization,
b) isolating positive clones, and
c) isolating the DNA-inserts from said clones.

2. The process according to claim 1, wherein the gene encodes human BMP-2 having the amino acid sequence given in claim 1.

3. A process for the preparation of a gene encoding a protein exhibiting properties of human BMP-2 and comprising a DNA sequence:
a) which differs from a DNA sequence of claim 1 in codon sequence due to the degeneracy of the genetic code;
b) which hybridizes with a DNA sequence of claim 1 or section (a), above; or
c) represents a fragment, allelic or other variation of a DNA sequence of claim 1, whether said variation results in changes in the peptide sequence or not,
wherein said process comprises standard techniques of molecular biology.

4. The process according to claim 3, wherein the DNA sequence is a genomic DNA sequence.

5. The process according to claim 3, wherein the DNA sequence is a cDNA sequence.

6. A process for the preparation of a gene encoding bovine BMP-2 comprising the following DNA sequence: 
(1)                    15                    30                    45
GGC CAC GAT GGG AAA GGA CAC CCT CTC CAC AGA AGA GAA AAG CGG
G   H   D   G   K   G   H   P   L   H   R   R   E   K   R

                    60                    75                    90
CAA GCA AAA CAC AAA CAG CGG AAA CGC CTC AAG TCC AGC TGT AAG
Q   A   K   H   K   Q   R   K   R   L   K   S   S   C   K

                    105                   120                   135
AGA CAC CCT TTA TAT GTG GAC TTC AGT GAT GTG GGG TGG AAT GAC
R   H   P   L   Y   V   D   F   S   D   V   G   W   N   D

                    150                   165                   180
TGG ATC GTT GCA CCG CCG GGG TAT CAT GCC TTT TAC TGC CAT GGG
W   I   V   A   P   P   G   Y   H   A   F   Y   C   H   G

                    195                   210                   225
GAG TGC CCT TTT CCC CTG GCC GAT CAC CTT AAC TCC ACG AAT CAT
E   C   P   F   P   L   A   D   H   L   N   S   T   N   H

                    240                   255                   270
GCC ATT CTC CAA ACT CTG GTC AAC TCA GTT AAC TCT AAG ATT CCC
A   I   V   Q   T   L   V   N   S   V   N   S   K   I   P

                    385                   300                   315
AAG GCA TGC TGT GTC CCA ACA GAG CTC AGC GCC ATC TCC ATG CTG
K   A   C   C   V   P   T   E   L   S   A   I   S   M   L

                    330                   345                   360
TAC CTT GAT GAG AAT GAG AAG GTG GTA TTA AAG AAC TAT CAG GAC
Y   L   D   E   N   E   K   V   V   L   K   N   Y   Q   D

                    375               (129)       397        407
ATG GTT GTC GAG GGT TGT GGG TGT CGT TAGCACAGCA AAATAAAATA
M   V   V   E   G   C   G   C   R

        417        427        437        447        457
TAAATATATA TATATATATA TTAGAAAAAC AGCAAAAAAA TCAAGTTGAC

        467        477        487        497        507
ACTTTAATAT TTCCCAATGA AGACTTTATT TATGGAATGG AATGGAGAAA

        517        527        537        547        557
AAGAAAAACA CAGCTATTTT GAAAACTATA TTTATATCTA CCGAAAAGAA

        567        577        587
GTTGGGAAAA CAAATATTTT AATCAGAGAA TTATT,
 wherein said process comprises the following steps:
a) screening a gene library constructed from bovine liver DNA or cDNA with a labelled probe designed on the basis of the amino acid sequence of a fragment of bBMP-2,
b) isolating positive clones, and
c) isolating the DNA-inserts from said clones.

7. The process according to claim 6, wherein the gene encodes bovine BMP-2 having the amino acid sequence of claim 6.

8. A process for the preparation of a gene encoding a protein exhibiting properties of bovine BMP-2 and comprising DNA sequences:
a) which differ from a DNA sequence of claim 7 in codon sequence due to the degeneracy of the genetic code;
b) which hybridize with a DNA sequence of claim 7 or section a), above; or
c) represent fragments, allelic or other variations of a DNA sequence of claim 7, whether said variations result in changes in the peptide sequence or not,
wherein said process comprises standard techniques of molecular biology.

9. The process according to claim 8, wherein the DNA sequence is a genomic DNA sequence.

10. The process according to claim 8, wherein the DNA sequence is a cDNA sequence.

11. A process for the preparation of a gene encoding human BMP-4 comprising the following DNA sequence: 
10 20 30 40 50 60 70
CTCTAGAGGG CAGAGGAGGA GGGAGGGAGG GAAGGAGCGC GGAGCCCGGC CCGGAAGCTA GGTGAGTGTG

80 90 100 110 120 130 140
GCATCCGAGC TGAGGGACGC GAGCCTGAGA CGCCGCTGCT GCTCCGGCTG AGTATCTAGC TTGTCTCCCC

150 160 170 180 190 200 210
GATGGGATTC CCGTCCAAGC TATCTCGAGC CTGCAGCGCC ACAGTCCCCG GCCCTCGCCC AGGTTCACTG

220 230 240 250 260 270 280
CAACCGTTCA GAGGTCCCCA GGAGCTGCTG CTGGCGAGCC CGCTACTGCA GGGACCTATG GAGCCATTCC

290 300 310 320 330 340 350
GTAGTGCCAT CCCGAGCAAC GCACTGCTGC AGCTTCCCTG AGCCTTTCCA GCAAGTTTGT TCAAGATTGG

360 370 380 390 400 (1)
CTGTCAAGAA TCATGGACTG TTATTATATG CCTTGTTTTC TGTCAAGACA CC ATG ATT CCT


417 432 447 462
GGT AAC CGA ATG CTG ATG GTC GTT TTA TTA TGC CAA GTC CTG CTA GGA GGC GCG


477 492 507
AGC CAT GCT AGT TTG ATA CCT GAG ACG GGG AAG AAA AAA GTC GCC GAG ATT CAG


522 537 552 567
GGC CAC GCG GGA GGA CGC CGC TCA GGG CAG AGC CAT GAG CTC CTG CGG GAC TTC


582 597 612 627
GAG GCG ACA CTT CTG CAG ATG TTT GGG CTG CGC CGC CGC CCG CAG CCT AGC AAG


642 657 672
AGT GCC GTC ATT CCG GAC TAC ATG CGG GAT CTT TAC CGG CTT CAG TCT GGG GAG


687 702 717 732
GAG GAG GAA GAG CAG ATC CAC AGC ACT GGT CTT GAG TAT CCT GAG CGC CCG GCC

 
747 762 777
AGC CGG GCC AAC ACC GTG AGG AGC TTC CAC CAC GAA GAA CAT CTG GAG AAC ATC


792 807 822 837
CCA GGG ACC AGT GAA AAC TCT GCT TTT CGT TTC CTC TTT AAC CTC AGC AGC ATC


852 867 882 897
CCT GAG AAC GAG GTG ATC TCC TCT GCA GAG CTT CGG CTC TTC CGG GAG CAG GTG


912 927 942
GAC CAG GGC CCT GAT TGG GAA AGG GGC TTC CAC CGT ATA AAC ATT TAT GAG GTT


957 972 987 1002
ATG AAG CCC CCA GCA GAA GTG GTG CCT GGG CAC CTC ATC ACA CGA CTA CTG GAC


1017 1032 1047
ACG AGA CTG GTC CAC CAC AAT GTG ACA CGG TGG GAA ACT TTT GAT GTG AGC CCT


1062 1077 1092 1107
GCG GTC CTT CGC TGG ACC CGG GAG AAG CAG CCA AAC TAT GGG CTA GCC ATT GAG


1122 1137 1152 1167
GTG ACT CAC CTC CAT CAG ACT CGG ACC CAC CAG GGC CAG CAT GTC AGG ATT AGC


1182 1197 1212
CGA TCG TTA CCT CAA GGG AGT GGG AAT TGG GCC CAG CTC CGG CCC CTC CTG GTC


1227 1242 1257 1272
ACC TTT GGC CAT GAT GGC CGG GGC CAT GCC TTG ACC CGA CGC CGG AGG GCC AAG


1287 1302 1317
CGT AGC CCT AAG CAT CAC TCA CAG CGG GCC AGG AAG AAG AAT AAG AAC TGC CGG


1332 1347 1362 1377
CGC CAC TCG CTC TAT GTG GAC TTC AGC GAT GTG GGC TGG AAT GAC TGG ATT GTG


1392 1407 1422 1437
GCC CCA CCA GGC TAC CAG GCC TTC TAC TGC CAT GGG GAC TGC CCC TTT CCA CTG


1452 1467 1482
GCT GAC CAC CTC AAC TCA ACC AAC CAT GCC ATT GTG CAG ACC CTG GTC AAT TCT

 
1497                    1512                    1527                    1542
GTC AAT TCC AGT ATC CCC AAA GCC TGT TGT GTG CCC ACT GAA CTG AGT GCC ATC


            1557                    1572                    1587
TCC ATG CTG TAC CTG GAT GAG TAT GAT AAG GTG GTA CTG AAA AAT TAT CAG GAG


1602                    1617            (408)     1636       1646       1656
ATG GTA GTA GAG GGA TGT GGG TGC CGC TGAGATCAGG CAGTCCTTGA GGATAGACAG


      1666       1676       1686       1696       1706       1716       1726
ATATACACAC CACACACACA CACCACATAC ACCACACACA CACGTTCCCA TCCACTCACC CACACACTAC

      1736       1746       1756       1766       1776       1786       1796
ACAGACTGCT TCCTTATAGC TGGACTTTTA TTTAAAAAAA AAAAAAAAAA AATGGAAAAA ATCCCTAAAC

      1806       1816       1826       1836       1846       1856       1866
ATTCACCTTG ACCTTATTTA TGACTTTACG TGCAAATGTT TTGACCATAT TGATCATATA TTTTGACAAA

      1876       1886       1896       1906       1916       1926       1936
ATATATTTAT AACTACGTAT TAAAAGAAAA AAATAAAATG AGTCATTATT TTAAAAAAAA AAAAAAAACT

      1946
CTAGAGTCGA CGGAATTC,
 wherein said process comprises the following steps:
a) screening of a gene library constructed from U-2 OS derived DNA or cDNA with a labelled bBMP-2 fragment by hybridization,
b) isolating positive clones, and
c) isolating the DNA-inserts from said clones.

12. The process according to claim 11, wherein the gene encodes human BMP-4 having the amino acid sequence given in claim 11.

13. A process for the preparation of a gene encoding a protein exhibiting properties of BMP-4 and comprising a DNA sequence:
a) which differs from a DNA sequence of claim 11 in codon sequence due to the degeneracy of the genetic code;
b) which hybridizes with DNA sequence of claim 11 or section a), above; or
c) represents a fragment, allelic or other variation of a DNA sequence of claim 11, whether said variation results in changes in the peptide sequence or not,
wherein said process comprises standard techniques of molecular biology.

14. The process according to claim 13, wherein the DNA sequence is a genomic DNA sequence.

15. The process according to claim 13, wherein the DNA sequence is a cDNA sequence.

16. A vector containing the gene or DNA sequence prepared according to any one of claims 1 to 15 in operative association with an expression control sequence.

17. A cell transformed with a vector of claim 16.

18. The cell of claim 17 which is a mammalian cell, a bacterial cell, an insect cell or a yeast cell.

19. The cell of claim 18 which is a CHO cell.

20. A process for the preparation of a protein exhibiting properties of BMP-2, wherein said process comprises the steps of culturing in a suitable culture medium a cell transformed with an expression vector comprising a gene or a DNA sequence prepared according to any one of claims 1 to 10, and recovering said protein from said culture medium.

21. A process for the preparation of a protein exhibiting properties of BMP-4, wherein said process comprises the steps of culturing in a suitable culture medium a cell transformed with an expression vector comprising a gene or a DNA sequence prepared according to any one of claims 11 to 15, and recovering said protein from said culture medium.

22. A process for producing a protein exhibiting properties of BMP-2 or BMP-4, comprising the steps of culturing in a suitable culture medium the cell of claim 17 and isolating said protein from said culture medium.

23. A process for the preparation of a pharmaceutical composition comprising combining the proteins prepared according to any one of claims 20 to 22, individually or in combination with a pharmaceutically acceptable vehicle.

24. The process according to claim 23, wherein said pharmaceutical composition further comprises a matrix capable of delivering the composition to the site of the bone or cartilage defect and providing a structure for inducing bone or cartilage formation.

25. The process according to claim 24, wherein said matrix comprises hydroxyapatite, collagen, polylactic acid or tricalcium phosphate.

26. Use of a protein prepared according to any one of claims 20 to 22, individually or in combination, for the preparation of a pharmaceutical composition for inducing bone or cartilage formation.

## Patentansprüche (Patentansprüche für folgende(n) Vertragsstaat(en): BE, CH, DE, FR, GB, IT, LI, LU, NL, SE)

1. Menschliches BMP-2 codierendes Gen, umfassend die nachfolgende DNA-Sequenz:

10 20 30 40 50
GTCGACTCTA GAGTGTGTGT CAGCACTTGG CTGGGGACTT CTTGAACTTG

60 70 80 90 100
CAGGGAGAAT AACTTGCGCA CCCCACTTTG CGCCGGTGCC TTTGCCCCAG

110 120 130 140 150
CGGAGCCTGC TTCGCCATCT CCGAGCCCCA CCGCCCCTCC ACTCCTCGGC

160 170 180 190 200
CTTGCCCGAC ACTGAGACGC TGTTCCCAGC GTGAAAAGAG AGACTGCGCG

210 220 230 240 250
GCCGGCACCC GGGAGAAGGA GGAGGCAAAG AAAAGGAACG GACATTCGGT

260 270 280 290 300
CCTTGCGCCA GGTCCTTTGA CCAGAGTTTT TCCATGTGGA CGCTCTTTCA

310 320 330 340 350
ATGGACGTGT CCCCGCGTGC TTCTTAGACG GACTGCGGTC TCCTAAAGGT

(1) 370 385 400
CGACC ATG GTG GCC GGG ACC CGC TGT CTT CTA GCG TTG CTG CTT CCC CAG GTC


415 430 445
CTC CTG GGC GGC GCG GCT GGC CTC GTT CCG GAG CTG GGC CGC AGG AAG TTC GCG


460 475 490 505
GCG GCG TCG TCG GGC CGC CCC TCA TCC CAG CCC TCT GAC GAG GTC CTG AGC GAG


520 535 550 565
TTC GAG TTG CGG CTG CTC AGC ATG TTC GGC CTG AAA CAG AGA CCC ACC CCC AGC


580 595 610
AGG GAC GCC GTG GTG CCC CCC TAC ATG CTA GAC CTG TAT CGC AGG CAC TCG GGT


625 640 655 670
CAG CCG GGC TCA CCC GCC CCA GAC CAC CGG TTG GAG AGG GCA GCC AGC CGA GCC


685 700 715
AAC ACT GTG CGC AGC TTC CAC CAT GAA GAA TCT TTG GAA GAA CTA CCA GAA ACG


730 745 760 775
AGT GGG AAA ACA ACC CGG AGA TTC TTC TTT AAT TTA AGT TCT ATC CCC ACG GAG


790 805 820 835
GAG TTT ATC ACC TCA GCA GAG CTT CAG GTT TTC CGA GAA CAG ATG CAA GAT GCT


850 865 880
TTA GGA AAC AAT AGC AGT TTC CAT CAC CGA ATT AAT ATT TAT GAA ATC ATA AAA


895 910 925 940
CCT GCA ACA GCC AAC TCG AAA TTC CCC GTG ACC AGT CTT TTG GAC ACC AGG TTG


955 970 985
GTG AAT CAG AAT GCA AGC AGG TGG GAA AGT TTT GAT GTC ACC CCC GCT GTG ATG


1000 1015 1030 1045
CGG TGG ACT GCA CAG GGA CAC GCC AAC CAT GGA TTC GTG GTG GAA GTG GCC CAC


1060 1075 1090 1105
TTG GAG GAG AAA CAA GGT GTC TCC AAG AGA CAT GTT AGG ATA AGC AGG TCT TTG


1120 1135 1150
CAC CAA GAT GAA CAC AGC TGG TCA CAG ATA AGG CCA TTG CTA GTA ACT TTT GGC


1165 1180 1195 1210
CAT GAT GGA AAA GGG CAT CCT CTC CAC AAA AGA GAA AAA CGT CAA GCC AAA CAC


1225 1240 1255
AAA CAG CGG AAA CGC CTT AAG TCC AGC TGT AAG AGA CAC CCT TTG TAC GTG GAC


1270 1285 1300 1315
TTC AGT GAC GTG GGG TGG AAT GAC TGG ATT GTG GCT CCC CCG GGG TAT CAC GCC


1330 1345 1360 1375
TTT TAC TGC CAC GGA GAA TGC CCT TTT CCT CTG GCT GAT CAT CTG AAC TCC ACT


1390 1405 1420
AAT CAT GCC ATT GTT CAG ACG TTG GTC AAC TCT GTT AAC TCT AAG ATT CCT AAG


1435 1450 1465 1480
GCA TGC TGT GTC CCG ACA GAA CTC AGT GCT ATC TCG ATG CTG TAC CTT GAC GAG


1495 1510 1525
AAT GAA AAG GTT GTA TTA AAG AAC TAT CAG GAC ATG GTT GTG GAG GGT TGT GGG


1540(396) 1553 1563 1573 1583 1593 1603
TGT CGC TAGTACAGCA AAATTAAATA CATAAATATA TATATATATA TATATTTTAG AAAAAAGAAA


AAAA

2. Gen, das menschliches BMP-2 codiert, das die in Anspruch 1 angegebene Aminosäuresequenz aufweist.

3. Gen, das ein Protein codiert, das Eigenschaften von menschlichem BMP-2 zeigt, und eine DNA-Sequenz umfaßt, die:
(a) sich in der Codonsequenz infolge der Degeneriertheit des genetischen Codes von einer DNA-Sequenz nach Anspruch 1 unterscheidet;
(b) mit einer DNA-Sequenz nach Anspruch 1 oder nach vorstehendem Absatz (a) hybridisiert; oder
(c) ein Fragment, eine allelische oder eine andere Variation einer DNA-Sequenz nach Anspruch 1 darstellt, unabhängig davon, ob die Variation zu Änderungen in der Peptidsequenz führt oder nicht.

4. DNA-Sequenz nach Anspruch 3, dadurch gekennzeichnet, daß sie eine genomische DNA-Sequenz ist.

5. DNA-Sequenz nach Anspruch 3, dadurch gekennzeichnet, daß sie eine cDNA-Sequenz ist.

6. Rinder-BMP-2 codierendes Gen, umfassend die nachfolgende DNA-Sequenz:

(1)                15                30                45
GGC CAC GAT GGG AAA GGA CAC CCT CTC CAC AGA AGA GAA AAG CGG
G   H   D   G   K   G   H   P   L   H   R   R   E   K   R

                    60                75                90
CAA GCA AAA CAC AAA CAG CGG AAA CGC CTC AAG TCC AGC TGT AAG
Q   A   K   H   K   Q   R   K   R   L   K   S   S   C   K

                    105               120               135
AGA CAC CCT TTA TAT GTG GAC TTC AGT GAT GTG GGG TGG AAT GAC
R   H   P   L   Y   V   D   F   S   D   V   G   W   N   D

                    150               165               180
TGG ATC GTT GCA CCG CCG GGG TAT CAT GCC TTT TAC TGC CAT GGG
W   I   V   A   P   P   G   Y   H   A   F   Y   C   H   G

                    195               210               225
GAG TGC CCT TTT CCC CTG GCC GAT CAC CTT AAC TCC ACG AAT CAT
E   C   P   F   P   L   A   D   H   L   N   S   T   N   H

                    240               255               270
GCC ATT CTC CAA ACT CTG GTC AAC TCA GTT AAC TCT AAG ATT CCC
A   I   V   Q   T   L   V   N   S   V   N   S   K   I   P

                    385               300               315
AAG GCA TGC TGT GTC CCA ACA GAG CTC AGC GCC ATC TCC ATG CTG
K   A   C   C   V   P   T   E   L   S   A   I   S   M   L

                    330               345               360
TAC CTT GAT GAG AAT GAG AAG GTG GTA TTA AAG AAC TAT CAG GAC
Y   L   D   E   N   E   K   V   V   L   K   N   Y   Q   D

                    375               (129)      397        407
ATG GTT GTC GAG GGT TGT GGG TGT CGT TAGCACAGCA AAATAAAATA
M   V   V   E   G   C   G   C   R

417 427 437 447 457
TAAATATATA TATATATATA TTAGAAAAAC AGCAAAAAAA TCAAGTTGAC

467 477 487 497 507
ACTTTAATAT TTCCCAATGA AGACTTTATT TATGGAATGG AATGGAGAAA

517 527 537 547 557
AAGAAAAACA CAGCTATTTT GAAAACTATA TTTATATCTA CCGAAAAGAA

567 577 587
GTTGGGAAAA CAAATATTTT AATCAGAGAA TTATT ,

7. Gen, das Rinder-BMP-2 codiert, das die Aminosäuresequenz von Anspruch 6 enthält.

8. Gen, das ein Protein codiert, das Eigenschaften von Rinder-BMP-2 zeigt, und DNA-Sequenzen umfaßt, die:
(a) sich in der Codonsequenz infolge der Degeneriertheit des genetischen Codes von einer DNA-Sequenz nach Anspruch 7 unterscheiden;
(b) mit einer DNA-Sequenz nach Anspruch 7 oder nach vorstehendem Absatz (a) hybridisieren; oder
(c) Fragmente, allelische oder andere Variationen einer DNA-Sequenz nach Anspruch 7 darstellen, unabhängig davon, ob die Variationen zu Änderungen in der Peptidsequenz führen oder nicht.

9. DNA-Sequenz nach Anspruch 8, dadurch gekennzeichnet, daß sie eine genomische DNA-Sequenz ist.

10. DNA-Sequenz nach Anspruch 8, dadurch gekennzeichnet, daß sie eine cDNA-Sequenz ist.

11. Menschliches BMP-4 codierendes Gen, umfassend die nachfolgende DNA-Sequenz:

10         20         30         40         50
CTCTAGAGGG CAGAGGAGGA GGGAGGGAGG GAAGGAGCGC GGAGCCCGGC

60 70 80 90 100
CCGGAAGCTA GGTGAGTGTG GCATCCGAGC TGAGGGACGC GAGCCTGAGA

110 120 130 140 150
CGCCGCTGCT GCTCCGGCTG AGTATCTAGC TTGTCTCCCC GATGGGATTC

160 170 180 190 200
CCGTCCAAGC TATCTCGAGC CTGCAGCGCC ACAGTCCCCG GCCCTCGCCC

210 220 230 240 250
AGGTTCACTG CAACCGTTCA GAGGTCCCCA GGAGCTGCTG CTGGCGAGCC

260 270 280 290 300
CGCTACTGCA GGGACCTATG GAGCCATTCC GTAGTGCCAT CCCGAGCAAC

310 320 330 340 350
GCACTGCTGC AGCTTCCCTG AGCCTTTCCA GCAAGTTTGT TCAAGATTGG

360 370 380 390 400
CTGTCAAGAA TCATGGACTG TTATTATATG CCTTGTTTTC TGTCAAGACA

(1)
CC ATG ATT CCT


417 432 447 462
GGT AAC CGA ATG CTG ATG GTC GTT TTA TTA TGC CAA GTC CTG CTA GGA GGC GCG


477 492 507
AGC CAT GCT AGT TTG ATA CCT GAG ACG GGG AAG AAA AAA GTC GCC GAG ATT CAG


522 537 552 567
GGC CAC GCG GGA GGA CGC CGC TCA GGG CAG AGC CAT GAG CTC CTG CGG GAC TTC


582 597 612 627
GAG GCG ACA CTT CTG CAG ATG TTT GGG CTG CGC CGC CGC CCG CAG CCT AGC AAG


642 657 672
AGT GCC GTC ATT CCG GAC TAC ATG CGG GAT CTT TAC CGG CTT CAG TCT GGG GAG


687 702 717 732
GAG GAG GAA GAG CAG ATC CAC AGC ACT GGT CTT GAG TAT CCT GAG CGC CCG GCC


747 762 777
AGC CGG GCC AAC ACC GTG AGG AGC TTC CAC CAC GAA GAA CAT CTG GAG AAC ATC


792 807 822 837
CCA GGG ACC AGT GAA AAC TCT GCT TTT CGT TTC CTC TTT AAC CTC AGC AGC ATC


852 867 882 897
CCT GAG AAC GAG GTG ATC TCC TCT GCA GAG CTT CGG CTC TTC CGG GAG CAG GTG


912 927 942
GAC CAG GGC CCT GAT TGG GAA AGG GGC TTC CAC CGT ATA AAC ATT TAT GAG GTT


957 972 987 1002
ATG AAG CCC CCA GCA GAA GTG GTG CCT GGG CAC CTC ATC ACA CGA CTA CTG GAC


1017 1032 1047
ACG AGA CTG GTC CAC CAC AAT GTG ACA CGG TGG GAA ACT TTT GAT GTG AGC CCT


1062 1077 1092 1107
GCG GTC CTT CGC TGG ACC CGG GAG AAG CAG CCA AAC TAT GGG CTA GCC ATT GAG


1122 1137 1152 1167
GTG ACT CAC CTC CAT CAG ACT CGG ACC CAC CAG GGC CAG CAT GTC AGG ATT AGC


1182 1197 1212
CGA TCG TTA CCT CAA GGG AGT GGG AAT TGG GCC CAG CTC CGG CCC CTC CTG GTC


1227 1242 1257 1272
ACC TTT GGC CAT GAT GGC CGG GGC CAT GCC TTG ACC CGA CGC CGG AGG GCC AAG


1287 1302 1317
CGT AGC CCT AAG CAT CAC TCA CAG CGG GCC AGG AAG AAG AAT AAG AAC TGC CGG


1332 1347 1362 1377
CGC CAC TCG CTC TAT GTG GAC TTC AGC GAT GTG GGC TGG AAT GAC TGG ATT GTG


1392 1407 1422 1437
GCC CCA CCA GGC TAC CAG GCC TTC TAC TGC CAT GGG GAC TGC CCC TTT CCA CTG


1452 1467 1482
GCT GAC CAC CTC AAC TCA ACC AAC CAT GCC ATT GTG CAG ACC CTG GTC AAT TCT


1497 1512 1527 1542
GTC AAT TCC AGT ATC CCC AAA GCC TGT TGT GTG CCC ACT GAA CTG AGT GCC ATC


1557 1572 1587
TCC ATG CTG TAC CTG GAT GAG TAT GAT AAG GTG GTA CTG AAA AAT TAT CAG GAG


1602 1617 (408) 1636 1646 1656
ATG GTA GTA GAG GGA TGT GGG TGC CGC TGAGATCAGG CAGTCCTTGA GGATAGACAG


1666 1676 1686 1696 1706
ATATACACAC CACACACACA CACCACATAC ACCACACACA CACGTTCCCA

1716 1726 1736 1746 1756
TCCACTCACC CACACACTAC ACAGACTGCT TCCTTATAGC TGGACTTTTA

1766 1776 1786 1796 1806
TTTAAAAAAA AAAAAAAAAA AATGGAAAAA ATCCCTAAAC ATTCACCTTG

1816 1826 1836 1846 1856
ACCTTATTTA TGACTTTACG TGCAAATGTT TTGACCATAT TGATCATATA

1866 1876 1886 1896 1906
TTTTGACAAA ATATATTTAT AACTACGTAT TAAAAGAAAA AAATAAAATG

1916 1926 1936 1946
AGTCATTATT TTAAAAAAAA AAAAAAAACT CTAGAGTCGA CGGAATTC

12. Gen, das menschliches BMP-4 codiert, das die in Anspruch 11 angegebene Aminosäuresequenz aufweist.

13. Gen, das ein Protein codiert, das Eigenschaften von BMP-4 zeigt, und eine DNA-Sequenz umfaßt, die:
(a) sich in der Codonsequenz infolge der Degeneriertheit des genetischen Codes von einer DNA-sequenz nach Anspruch 11 unterscheidet;
(b) mit einer DNA-Sequenz nach Anspruch 11 oder nach vorstehendem Absatz (a) hybridisiert; oder
(c) ein Fragment, eine allelische oder eine andere Variation einer DNA-Sequenz nach Anspruch 11 darstellt, unabhängig davon, ob die Variation zu Änderungen in der Peptidsequenz führt oder nicht.

14. DNA-Sequenz nach Anspruch 13, dadurch gekennzeichnet, daß sie eine genomische DNA-Sequenz ist.

15. DNA-Sequenz nach Anspruch 13, dadurch gekennzeichnet, daß sie eine cDNA-Sequenz ist.

16. Vektor, enthaltend das Gen oder die DNA-Sequenz nach einem der Ansprüche 1 bis 15 in einer funktionellen Verbindung mit einer Expressions-Kontrollsequenz.

17. Zelle, dadurch gekennzeichnet, daß sie mit einem Vektor nach Anspruch 16 transformiert ist.

18. Zelle nach Anspruch 17, dadurch gekennzeichnet, daß sie eine Säugerzelle, eine Bakterienzelle, eine Insektenzelle oder eine Hefezelle ist.

19. Zelle nach Anspruch 18, dadurch gekennzeichnet, daß sie eine CHO-Zelle ist.

20. Protein, das Eigenschaften von BMP-2 aufweist, das durch ein Gen oder eine DNA-Sequenz nach einem der Ansprüche 1 bis 10 codiert ist.

21. Protein, das Eigenschaften von BMP-2 aufweist, das erhältlich ist durch die Schritte
- Züchten einer mit einem Expressionsvektor transformierten Zelle in einem geeigneten Kulturmedium, wobei der Vektor ein Gen oder eine DNA-Sequenz nach einem der Ansprüche 1 bis 10 umfaßt, und
- Gewinnen des Proteins aus dem Kulturmedium.

22. Protein, das Eigenschaften von BMP-4 aufweist, das durch ein Gen oder eine DNA-Sequenz nach einem der Ansprüche 11 bis 15 codiert ist.

23. Protein, das Eigenschaften von BMP-4 aufweist, das erhältlich ist durch die Schritte
- Züchten einer mit einem Expresionsvektor transformierten Zelle in einem geeigneten Kulturmedium, wobei der Vektor ein Gen oder eine DNA-Sequenz nach einem der Ansprüche 11 bis 15 umfaßt und
- Isolieren des Proteins aus dem Kulturmedium.

24. Verfahren zur Herstellung des Proteins nach Anspruch 21 oder 23, umfassend die Schritte
- Züchten der Zelle nach Anspruch 17 in einem geeigneten Kulturmedium und
- Gewinnen des Proteins aus dem Kulturmedium.

25. Arzneimittel, dadurch gekennzeichnet, daß es, einzeln oder in Kombination, die Proteine nach einem der Ansprüche 20 bis 23 und einen pharmakologisch verträglichen Träger umfaßt.

26. Arzneimittel nach Anspruch 25, dadurch gekennzeichnet, daß es ferner eine Matrix umfaßt, die fähig ist, das Arzneimittel an die Stelle des Knochen- oder Knorpelschadens zu liefern und eine Struktur zur Induktion der Knochen- oder Knorpelbildung bereitzustellen.

27. Arzneimittel nach Anspruch 26, dadurch gekennzeichnet, daß die Matrix Hydroxyapatit, Kollagen, Polyessigsäure oder Tricalciumphosphat umfaßt.

28. Verwendung des Proteins nach einem der Ansprüche 20 bis 23, einzeln oder in Kombination, zur Herstellung eines Arzneimittels zur Induktion der Knochen- oder Knorpelbildung.

## Patentansprüche (Patentansprüche für folgende(n) Vertragsstaat(en): AT)

1. Verfahren zur Herstellung eines menschliches BMP-2 codierenden Gens, das die nachfolgende DNA-Sequenz umfaßt: 
10 20 30 40 50
GTCGACTCTA GAGTGTGTGT CAGCACTTGG CTGGGGACTT CTTGAACTTG

60 70 80 90 100
CAGGGAGAAT AACTTGCGCA CCCCACTTTG CGCCGGTGCC TTTGCCCCAG

110 120 130 140 150
CGGAGCCTGC TTCGCCATCT CCGAGCCCCA CCGCCCCTCC ACTCCTCGGC

160 170 180 190 200
CTTGCCCGAC ACTGAGACGC TGTTCCCAGC GTGAAAAGAG AGACTGCGCG

210 220 230 240 250
GCCGGCACCC GGGAGAAGGA GGAGGCAAAG AAAAGGAACG GACATTCGGT

260 270 280 290 300
CCTTGCGCCA GGTCCTTTGA CCAGAGTTTT TCCATGTGGA CGCTCTTTCA

310 320 330 340 350
ATGGACGTGT CCCCGCGTGC TTCTTAGACG GACTGCGGTC TCCTAAAGGT

(1) 370 385 400
CGACC ATG GTG GCC GGG ACC CGC TGT CTT CTA GCG TTG CTG CTT CCC CAG GTC


415 430 445
CTC CTG GGC GGC GCG GCT GGC CTC GTT CCG GAG CTG GGC CGC AGG AAG TTC GCG


460 475 490 505
GCG GCG TCG TCG GGC CGC CCC TCA TCC CAG CCC TCT GAC GAG GTC CTG AGC GAG


520 535 550 565
TTC GAG TTG CGG CTG CTC AGC ATG TTC GGC CTG AAA CAG AGA CCC ACC CCC AGC


580 595 610
AGG GAC GCC GTG GTG CCC CCC TAC ATG CTA GAC CTG TAT CGC AGG CAC TCG GGT

 
625 640 655 670
CAG CCG GGC TCA CCC GCC CCA GAC CAC CGG TTG GAG AGG GCA GCC AGC CGA GCC


685 700 715
AAC ACT GTG CGC AGC TTC CAC CAT GAA GAA TCT TTG GAA GAA CTA CCA GAA ACG


730 745 760 775
AGT GGG AAA ACA ACC CGG AGA TTC TTC TTT AAT TTA AGT TCT ATC CCC ACG GAG


790 805 820 835
GAG TTT ATC ACC TCA GCA GAG CTT CAG GTT TTC CGA GAA CAG ATG CAA GAT GCT


850 865 880
TTA GGA AAC AAT AGC AGT TTC CAT CAC CGA ATT AAT ATT TAT GAA ATC ATA AAA


895 910 925 940
CCT GCA ACA GCC AAC TCG AAA TTC CCC GTG ACC AGT CTT TTG GAC ACC AGG TTG


955 970 985
GTG AAT CAG AAT GCA AGC AGG TGG GAA AGT TTT GAT GTC ACC CCC GCT GTG ATG


1000 1015 1030 1045
CGG TGG ACT GCA CAG GGA CAC GCC AAC CAT GGA TTC GTG GTG GAA GTG GCC CAC


1060 1075 1090 1105
TTG GAG GAG AAA CAA GGT GTC TCC AAG AGA CAT GTT AGG ATA AGC AGG TCT TTG


1120 1135 1150
CAC CAA GAT GAA CAC AGC TGG TCA CAG ATA AGG CCA TTG CTA GTA ACT TTT GGC


1165 1180 1195 1210
CAT GAT GGA AAA GGG CAT CCT CTC CAC AAA AGA GAA AAA CGT CAA GCC AAA CAC


1225 1240 1255
AAA CAG CGG AAA CGC CTT AAG TCC AGC TGT AAG AGA CAC CCT TTG TAC GTG GAC


1270 1285 1300 1315
TTC AGT GAC GTG GGG TGG AAT GAC TGG ATT GTG GCT CCC CCG GGG TAT CAC GCC


1330 1345 1360 1375
TTT TAC TGC CAC GGA GAA TGC CCT TTT CCT CTG GCT GAT CAT CTG AAC TCC ACT


1390 1405 1420
AAT CAT GCC ATT GTT CAG ACG TTG GTC AAC TCT GTT AAC TCT AAG ATT CCT AAG

 
1435 1450 1465 1480
GCA TGC TGT GTC CCG ACA GAA CTC AGT GCT ATC TCG ATG CTG TAC CTT GAC GAG


1495 1510 1525
AAT GAA AAG GTT GTA TTA AAG AAC TAT CAG GAC ATG GTT GTG GAG GGT TGT GGG


1540(396) 1553 1563 1573 1583 1593 1603
TGT CGC TAGTACAGCA AAATTAAATA CATAAATATA TATATATATA TATATTTTAG AAAAAAGAAA


AAAA,
 wobei das Verfahren die nachfolgenden Schritte umfaßt:
(a) Absuchen einer Genbank durch Hybridisieren mit einem markierten bBMP-2-Fragment, wobei die Genbank aus einer von U-2 OS abgeleiteten DNA oder cDNA konstruiert war,
(b) Isolieren positiver Clone und
(c) Isolieren der DNA-Insertionen aus diesen Clonen.

2. Verfahren nach Anspruch 1, dadurch gekennzeichnet, daß das Gen menschliches BMP-2 codiert, das die in Anspruch 1 angegebene Aminosäuresequenz aufweist.

3. Verfahren zur Herstellung eines Gens, das ein Protein codiert, das Eigenschaften von menschlichem BMP-2 zeigt, und eine DNA-Sequenz umfaßt, die:
(a) sich in der Codonsequenz infolge der Degeneriertheit des genetischen Codes von einer DNA-Sequenz nach Anspruch 1 unterscheidet;
(b) mit einer DNA-Sequenz nach Anspruch 1 oder nach vorstehendem Absatz (a) hybridisiert; oder
(c) ein Fragment, eine allelische oder eine andere Variation einer DNA-Sequenz nach Anspruch 1 darstellt, unabhängig davon, ob die Variation zu Änderungen in der Peptidsequenz führt oder nicht,
wobei das Verfahren Standardtechniken der Molekularbiologie umfaßt.

4. Verfahren nach Anspruch 3, dadurch gekennzeichnet, daß die DNA-Sequenz eine genomische DNA-Sequenz ist.

5. Verfahren nach Anspruch 3, dadurch gekennzeichnet, daß die DNA-Sequenz eine cDNA-Sequenz ist.

6. Verfahren zur Herstellung eines Rinder-BMP-2 codierenden Gens, umfassend die nachfolgende DNA-Sequenz: 
(1)                    15                    30                    45
GGC CAC GAT GGG AAA GGA CAC CCT CTC CAC AGA AGA GAA AAG CGG
G   H   D   G   K   G   H   P   L   H   R   R   E   K   R

                       60                    75                    90
CAA GCA AAA CAC AAA CAG CGG AAA CGC CTC AAG TCC AGC TGT AAG
Q   A   K   H   K   Q   R   K   R   L   K   S   S   C   K

                       105                   120                   135
AGA CAC CCT TTA TAT GTG GAC TTC AGT GAT GTG GGG TGG AAT GAC
R   H   P   L   Y   V   D   F   S   D   V   G   W   N   D

                       150                   165                   180
TGG ATC GTT GCA CCG CCG GGG TAT CAT GCC TTT TAC TGC CAT GGG
W   I   V   A   P   P   G   Y   H   A   F   Y   C   H   G

                       195                   210                   225
GAG TGC CCT TTT CCC CTG GCC GAT CAC CTT AAC TCC ACG AAT CAT
E   C   P   F   P   L   A   D   H   L   N   S   T   N   H

                       240                   255                   270
GCC ATT CTC CAA ACT CTG GTC AAC TCA GTT AAC TCT AAG ATT CCC
A   I   V   Q   T   L   V   N   S   V   N   S   K   I   P

                       385                   300                   315
AAG GCA TGC TGT GTC CCA ACA GAG CTC AGC GCC ATC TCC ATG CTG
K   A   C   C   V   P   T   E   L   S   A   I   S   M   L

                       330                   345                   360
TAC CTT GAT GAG AAT GAG AAG GTG GTA TTA AAG AAC TAT CAG GAC
Y   L   D   E   N   E   K   V   V   L   K   N   Y   Q   D

                       375                   (129)      397        407
ATG GTT GTC GAG GGT TGT GGG TGT CGT TAGCACAGCA AAATAAAATA
M   V   V   E   G   C   G   C   R
 
417 427 437 447 457
TAAATATATA TATATATATA TTAGAAAAAC AGCAAAAAAA TCAAGTTGAC

467 477 487 497 507
ACTTTAATAT TTCCCAATGA AGACTTTATT TATGGAATGG AATGGAGAAA

517 527 537 547 557
AAGAAAAACA CAGCTATTTT GAAAACTATA TTTATATCTA CCGAAAAGAA

567 577 587
GTTGGGAAAA CAAATATTTT AATCAGAGAA TTATT
 wobei das Verfahren die nachfolgenden Schritte umfaßt:
(a) Absuchen einer Genbank mit einer markierten auf der Grundlage der Aminosäuresequenz eines Fragmentes von bBMP-2 entworfenen Sonde, wobei die Genbank aus Rinderleber-DNA oder cDNA konstruiert wurde,
(b) Isolieren positiver Clone und
(c) Isolieren der DNA-Insertionen aus diesen Clonen.

7. Verfahren nach Anspruch 6, dadurch gekennzeichnet, daß das Gen Rinder-BMP-2 codiert, das die Aminosäuresequenz von Anspruch 6 aufweist.

8. Verfahren zur Herstellung eines Genes, das ein Protein codiert, das Eigenschaften von Rinder-BMP-2 zeigt, und DNA-Sequenzen umfaßt, die:
(a) sich in der Codonsequenz infolge der Degeneriertheit des genetischen Codes von einer DNA-Sequenz nach Anspruch 7 unterscheiden;
(b) mit einer DNA-Sequenz nach Anspruch 7 oder nach vorstehendem Absatz (a) hybridisieren; oder
(c) Fragmente, allelische oder andere Variationen einer DNA-Sequenz nach Anspruch 7 darstellen, unabhängig davon, ob die Variationen zu Änderungen in der Peptidsequenz führen oder nicht,
wobei das Verfahren Standardtechniken der Molekularbiologie umfaßt.

9. Verfahren nach Anspruch 8, dadurch gekennzeichnet, daß die DNA-Sequenz eine genomische DNA-Sequenz ist.

10. Verfahren nach Anspruch 8, dadurch gekennzeichnet, daß die DNA-Sequenz eine cDNA-Sequenz ist.

11. Verfahren zur Herstellung eines menschliches BMP-4 codierenden Genes, das die nachfolgende DNA-Sequenz umfaßt: 
10 20 30 40 50
CTCTAGAGGG CAGAGGAGGA GGGAGGGAGG GAAGGAGCGC GGAGCCCGGC

60 70 80 90 100
CCGGAAGCTA GGTGAGTGTG GCATCCGAGC TGAGGGACGC GAGCCTGAGA

110 120 130 140 150
CGCCGCTGCT GCTCCGGCTG AGTATCTAGC TTGTCTCCCC GATGGGATTC

160 170 180 190 200
CCGTCCAAGC TATCTCGAGC CTGCAGCGCC ACAGTCCCCG GCCCTCGCCC

210 220 230 240 250
AGGTTCACTG CAACCGTTCA GAGGTCCCCA GGAGCTGCTG CTGGCGAGCC

260 270 280 290 300
CGCTACTGCA GGGACCTATG GAGCCATTCC GTAGTGCCAT CCCGAGCAAC

310 320 330 340 350
GCACTGCTGC AGCTTCCCTG AGCCTTTCCA GCAAGTTTGT TCAAGATTGG

360 370 380 390 400
CTGTCAAGAA TCATGGACTG TTATTATATG CCTTGTTTTC TGTCAAGACA
 
(1)
CC ATG ATT CCT
   

417            432            447            462
GGT AAC CGA ATG CTG ATG GTC GTT TTA TTA TGC CAA GTC CTG CTA GGA GGC GCG


    477            492            507
AGC CAT GCT AGT TTG ATA CCT GAG ACG GGG AAG AAA AAA GTC GCC GAG ATT CAG


522            537            552            567
GGC CAC GCG GGA GGA CGC CGC TCA GGG CAG AGC CAT GAG CTC CTG CGG GAC TTC


    582            597            612            627
GAG GCG ACA CTT CTG CAG ATG TTT GGG CTG CGC CGC CGC CCG CAG CCT AGC AAG


        642            657            672
AGT GCC GTC ATT CCG GAC TAC ATG CGG GAT CTT TAC CGG CTT CAG TCT GGG GAG


    687            702            717            732
GAG GAG GAA GAG CAG ATC CAC AGC ACT GGT CTT GAG TAT CCT GAG CGC CCG GCC


        747            762            777
AGC CGG GCC AAC ACC GTG AGG AGC TTC CAC CAC GAA GAA CAT CTG GAG AAC ATC


792            807            822            837
CCA GGG ACC AGT GAA AAC TCT GCT TTT CGT TTC CTC TTT AAC CTC AGC AGC ATC


    852            867            882            897
CCT GAG AAC GAG GTG ATC TCC TCT GCA GAG CTT CGG CTC TTC CGG GAG CAG GTG


        912            927            942
GAC CAG GGC CCT GAT TGG GAA AGG GGC TTC CAC CGT ATA AAC ATT TAT GAG GTT


    957            972            987            1002
ATG AAG CCC CCA GCA GAA GTG GTG CCT GGG CAC CTC ATC ACA CGA CTA CTG GAC


        1017           1032           1047
ACG AGA CTG GTC CAC CAC AAT GTG ACA CGG TGG GAA ACT TTT GAT GTG AGC CCT


1062           1077           1092           1107
GCG GTC CTT CGC TGG ACC CGG GAG AAG CAG CCA AAC TAT GGG CTA GCC ATT GAG

 
1122                    1137                    1152                    1167
GTG ACT CAC CTC CAT CAG ACT CGG ACC CAC CAG GGC CAG CAT GTC AGG ATT AGC


                1182                    1197                    1212
CGA TCG TTA CCT CAA GGG AGT GGG AAT TGG GCC CAG CTC CGG CCC CTC CTG GTC


1227                    1242                    1257                    1272
ACC TTT GGC CAT GAT GGC CGG GGC CAT GCC TTG ACC CGA CGC CGG AGG GCC AAG


                1287                    1302                    1317
CGT AGC CCT AAG CAT CAC TCA CAG CGG GCC AGG AAG AAG AAT AAG AAC TGC CGG


1332                    1347                    1362                    1377
CGC CAC TCG CTC TAT GTG GAC TTC AGC GAT GTG GGC TGG AAT GAC TGG ATT GTG


        1392                    1407                    1422                    1437
GCC CCA CCA GGC TAC CAG GCC TTC TAC TGC CAT GGG GAC TGC CCC TTT CCA CTG


                1452                    1467                    1482
GCT GAC CAC CTC AAC TCA ACC AAC CAT GCC ATT GTG CAG ACC CTG GTC AAT TCT


    1497                    1512                    1527                    1542
GTC AAT TCC AGT ATC CCC AAA GCC TGT TGT GTG CCC ACT GAA CTG AGT GCC ATC


            1557                    1572                    1587
TCC ATG CTG TAC CTG GAT GAG TAT GAT AAG GTG GTA CTG AAA AAT TAT CAG GAG


1602                    1617            (408)       1636        1646        1656
ATG GTA GTA GAG GGA TGT GGG TGC CGC TGAGATCAGG CAGTCCTTGA GGATAGACAG


      1666        1676        1686        1696        1706
ATATACACAC CACACACACA CACCACATAC ACCACACACA CACGTTCCCA

      1716        1726        1736        1746        1756
TCCACTCACC CACACACTAC ACAGACTGCT TCCTTATAGC TGGACTTTTA

      1766        1776        1786        1796        1806
TTTAAAAAAA AAAAAAAAAA AATGGAAAAA ATCCCTAAAC ATTCACCTTG
 
1816 1826 1836 1846 1856
ACCTTATTTA TGACTTTACG TGCAAATGTT TTGACCATAT TGATCATATA

1866 1876 1886 1896 1906
TTTTGACAAA ATATATTTAT AACTACGTAT TAAAAGAAAA AAATAAAATG

1916 1926 1936 1946
AGTCATTATT TTAAAAAAAA AAAAAAAACT CTAGAGTCGA CGGAATTC
 wobei das Verfahren die nachfolgenden Schritte umfaßt:
(a) Absuchen einer Genbank durch Hybridisieren mit einem markierten bBMP-2-Fragment, wobei die Genbank aus einer von U-2 OS abgeleiteten DNA oder cDNA konstruiert war,
(b) Isolieren positiver Clone und
(c) Isolieren der DNA-Insertionen aus diesen Clonen.

12. Verfahren nach Anspruch 11, dadurch gekennzeichnet, daß das Gen menschliches BMP-4 codiert, das die in Anspruch 11 angegebene Aminosäuresequenz aufweist.

13. Verfahren zur Herstellung eines Genes, das ein Protein codiert, das Eigenschaften von BMP-4 zeigt, und eine DNA-Sequenz umfaßt, die:
(a) sich in der Codonsequenz infolge der Degeneriertheit des genetischen Codes von einer DNA-Sequenz nach Anspruch 11 unterscheidet;
(b) mit einer DNA-Sequenz nach Anspruch 11 oder vorstehendem Absatz (a) hybridisiert; oder
(c) ein Fragment, eine allelische oder eine andere Variation einer DNA-Sequenz nach Anspruch 11 darstellt, unabhängig davon, ob die Variation zu Änderungen in der Peptidsequenz führt oder nicht,
wobei das Verfahren Standardtechniken der Molekularbiologie umfaßt.

14. Verfahren nach Anspruch 13, dadurch gekennzeichnet, daß die DNA-Sequenz eine genomische DNA-Sequenz ist.

15. Verfahren nach Anspruch 13, dadurch gekennzeichnet, daß die DNA-Sequenz eine cDNA-Sequenz ist.

16. Vektor, enthaltend das Gen oder die DNA-Sequenz nach einem der Ansprüche 1 bis 15 in einer funktionellen Verbindung mit einer Expressions-Kontrollsequenz.

17. Zelle, dadurch gekennzeichnet, daß sie mit einem Vektor nach Anspruch 16 transformiert ist.

18. Zelle nach Anspruch 17, dadurch gekennzeichnet, daß sie eine Säugerzelle, eine Bakterienzelle, eine Insektenzelle oder eine Hefezelle ist.

19. Zelle nach Anspruch 18, dadurch gekennzeichnet, daß sie eine CHO-Zelle ist.

20. Verfahren zur Herstellung eines Proteins, das Eigenschaften von BMP-2 zeigt, umfassend die Schritte
- Züchten einer mit einem Expressionsvektor transformierten Zelle in einem geeigneten Kulturmedium, wobei der Expressionsvektor ein Gen oder eine DNA-Sequenz umfaßt, die nach einem der Ansprüche 1 bis 10 hergestellt wurden, und
- Gewinnen des Proteins aus dem Kulturmedium.

21. Verfahren zur Herstellung eines Proteins, das Eigenschaften von BMP-4 zeigt, umfassend die Schritte
- Züchten einer mit einem Expressionsvektor transformierten Zelle in einem geeigneten Kulturmedium, wobei der Expressionsvektor ein Gen oder eine DNA-Sequenz umfaßt, die nach einem der Ansprüche 11 bis 15 hergestellt wurden, und
- Gewinnen des Proteins aus dem Kulturmedium.

22. Verfahren zur Herstellung eines Proteins, das Eigenschaften von BMP-2 oder BMP-4 zeigt, umfassend die Schritte
- Züchten der Zelle nach Anspruch 17 in einem geeigneten Kulturmedium und
- Isolieren des Proteins aus dem Kulturmedium.

23. Verfahren zur Herstellung eines Arzneimittels, dadurch gekennzeichnet, daß es ein Kombinieren der nach einem der Ansprüche 20 bis 22 hergestellten Proteine, einzeln oder in Kombination, mit einem pharmakologisch verträglichen Träger umfaßt.

24. Verfahren nach Anspruch 23, dadurch gekennzeichnet, daß das Arzneimittel ferner eine Matrix umfaßt, die fähig ist, das Arzneimittel an die Stelle des Knochen- oder Knorpelschadens zu liefern und eine Struktur zur Induktion der Knochen- oder Knorpelbildung bereitzustellen.

25. Verfahren nach Anspruch 24, dadurch gekennzeichnet, daß die Matrix Hydroxyapatit, Kollagen, Polyessigsäure oder Tricalciumphosphat umfaßt.

26. Verwendung eines Proteins nach einem der Ansprüche 20 bis 22, einzeln oder in Kombination, zur Herstellung eines Arzneimittels zur Induktion der Knochen- oder Knorpelbildung.

## Revendications (Revendications pour l'(les) Etat(s) contractant(s) suivant(s): BE, CH, DE, FR, GB, IT, LI, LU, NL, SE)

1. Gène codant pour la BMP-2 humaine comprenant la séquence d'ADN suivante :

10 20 30 40 50 60 70
GTCGACTCTA GAGTGTGTGT CAGCACTTGG CTGGGGACTT CTTGAACTTG CAGGGAGAAT AACTTGCGCA

80 90 100 110 120 130 140
CCCCACTTTG CGCCGGTGCC TTTGCCCCAG CGGAGCCTGC TTCGCCATCT CCGAGCCCCA CCGCCCCTCC

150 160 170 180 190 200 210
ACTCCTCGGC CTTGCCCGAC ACTGAGACGC TGTTCCCAGC GTGAAAAGAG AGACTGCGCG GCCGGCACCC

220 230 240 250 260 270 280
GGGAGAAGGA GGAGGCAAAG AAAAGGAACG GACATTCGGT CCTTGCGCCA GGTCCTTTGA CCAGAGTTTT

290 300 310 320 330 340 350
TCCATGTGGA CGCTCTTTCA ATGGACGTGT CCCCGCGTGC TTCTTAGACG GACTGCGGTC TCCTAAAGGT

(1) 370 385 400
CGACC ATG GTG GCC GGG ACC CGC TGT CTT CTA GCG TTG CTG CTT CCC CAG GTC


415 430 445
CTC CTG GGC GGC GCG GCT GGC CTC GTT CCG GAG CTG GGC CGC AGG AAG TTC GCG


460 475 490 505
GCG GCG TCG TCG GGC CGC CCC TCA TCC CAG CCC TCT GAC GAG GTC CTG AGC GAG


520 535 550 565
TTC GAG TTG CGG CTG CTC AGC ATG TTC GGC CTG AAA CAG AGA CCC ACC CCC AGC


580 595 610
AGG GAC GCC GTG GTG CCC CCC TAC ATG CTA GAC CTG TAT CGC AGG CAC TCG GGT


625 640 655 670
CAG CCG GGC TCA CCC GCC CCA GAC CAC CGG TTG GAG AGG GCA GCC AGC CGA GCC


685 700 715
AAC ACT GTG CGC AGC TTC CAC CAT GAA GAA TCT TTG GAA GAA CTA CCA GAA ACG


730 745 760 775
AGT GGG AAA ACA ACC CGG AGA TTC TTC TTT AAT TTA AGT TCT ATC CCC ACG GAG


790 805 820 835
GAG TTT ATC ACC TCA GCA GAG CTT CAG GTT TTC CGA GAA CAG ATG CAA GAT GCT


850 865 880
TTA GGA AAC AAT AGC AGT TTC CAT CAC CGA ATT AAT ATT TAT GAA ATC ATA AAA


895 910 925 940
CCT GCA ACA GCC AAC TCG AAA TTC CCC GTG ACC AGT CTT TTG GAC ACC AGG TTG


955 970 985
GTG AAT CAG AAT GCA AGC AGG TGG GAA AGT TTT GAT GTC ACC CCC GCT GTG ATG


1000 1015 1030 1045
CGG TGG ACT GCA CAG GGA CAC GCC AAC CAT GGA TTC GTG GTG GAA GTG GCC CAC


1060 1075 1090 1105
TTG GAG GAG AAA CAA GGT GTC TCC AAG AGA CAT GTT AGG ATA AGC AGG TCT TTG


1120 1135 1150
CAC CAA GAT GAA CAC AGC TGG TCA CAG ATA AGG CCA TTG CTA GTA ACT TTT GGC


1165 1180 1195 1210
CAT GAT GGA AAA GGG CAT CCT CTC CAC AAA AGA GAA AAA CGT CAA GCC AAA CAC


1225 1240 1255
AAA CAG CGG AAA CGC CTT AAG TCC AGC TGT AAG AGA CAC CCT TTG TAC GTG GAC


1270 1285 1300 1315
TTC AGT GAC GTG GGG TGG AAT GAC TGG ATT GTG GCT CCC CCG GGG TAT CAC GCC


1330 1345 1360 1375
TTT TAC TGC CAC GGA GAA TGC CCT TTT CCT CTG GCT GAT CAT CTG AAC TCC ACT


1390 1405 1420
AAT CAT GCC ATT GTT CAG ACG TTG GTC AAC TCT GTT AAC TCT AAG ATT CCT AAG


1435                    1450                    1465                    1480
GCA TGC TGT GTC CCG ACA GAA CTC AGT GCT ATC TCG ATG CTG TAC CTT GAC GAG


        1495                    1510                    1525
AAT GAA AAG GTT GTA TTA AAG AAC TAT CAG GAC ATG GTT GTG GAG GGT TGT GGG


1540(396)       1553        1563        1573        1583        1593        1603
TGT CGC TAGTACAGCA AAATTAAATA CATAAATATA TATATATATA TATATTTTAG AAAAAAGAAA


AAAA

2. Gène codant pour la BMP-2 humaine comportant la séquence d'acides aminés donnée à la revendication 1.

3. Gène codant pour une protéine montrant des propriétés de la BMP-2 humaine et comprenant une séquence d'ADN :
(a) qui diffère d'une séquence d'ADN de la revendication 1 dans la séquence de codons du fait de la dégénérescence du code génétique ;
(b) qui s'hybride avec une séquence d'ADN de la revendication 1 ou du paragraphe (a) ci-dessus ; ou
(c) représente un fragment, une variation allélique ou autre d'une séquence d'ADN de la revendication 1, que cette variation résulte de changements dans la séquence peptidique ou non.

4. Séquence d'ADN suivant la revendication 3, qui est une séquence d'ADN génomique.

5. Séquence d'ADN suivant la revendication 3, qui est une séquence d'ADNc.

6. Gène codant pour la BMP-2 bovine comprenant la séquence d'ADN suivante :

(1)                    15                    30                    45
GGC CAC GAT GGG AAA GGA CAC CCT CTC CAC AGA AGA GAA AAG CGG
G   H   D   G   K   G   H   P   L   H   R   R   E   K   R

                       60                    75                    90
CAA GCA AAA CAC AAA CAG CGG AAA CGC CTC AAG TCC AGC TGT AAG
Q   A   K   H   K   Q   R   K   R   L   K   S   S   C   K

                       105                   120                   135
AGA CAC CCT TTA TAT GTG GAC TTC AGT GAT GTG GGG TGG AAT GAC
R   H   P   L   Y   V   D   F   S   D   V   G   W   N   D

                       150                   165                   180
TGG ATC GTT GCA CCG CCG GGG TAT CAT GCC TTT TAC TGC CAT GGG
W   I   V   A   P   P   G   Y   H   A   F   Y   C   H   G

                       195                   210                   225
GAG TGC CCT TTT CCC CTG GCC GAT CAC CTT AAC TCC ACG AAT CAT
E   C   P   F   P   L   A   D   H   L   N   S   T   N   H

                       240                   255                   270
GCC ATT CTC CAA ACT CTG GTC AAC TCA GTT AAC TCT AAG ATT CCC
A   I   V   Q   T   L   V   N   S   V   N   S   K   I   P

                       385                   300                   315
AAG GCA TGC TGT GTC CCA ACA GAG CTC AGC GCC ATC TCC ATG CTG
K   A   C   C   V   P   T   E   L   S   A   I   S   M   L

                       330                   345                   360
TAC CTT GAT GAG AAT GAG AAG GTG GTA TTA AAG AAC TAT CAG GAC
Y   L   D   E   N   E   K   V   V   L   K   N   Y   Q   D

                       375               (129)       397        407
ATG GTT GTC GAG GGT TGT GGG TGT CGT TAGCACAGCA AAATAAAATA
M   V   V   E   G   C   G   C   R

       417        427        437        447        457
TAAATATATA TATATATATA TTAGAAAAAC AGCAAAAAAA TCAAGTTGAC

       467        477        487        497        507
ACTTTAATAT TTCCCAATGA AGACTTTATT TATGGAATGG AATGGAGAAA

       517        527        537        547        557
AAGAAAAACA CAGCTATTTT GAAAACTATA TTTATATCTA CCGAAAAGAA

       567        577        587
GTTGGGAAAA CAAATATTTT AATCAGAGAA TTATT

7. Gène codant pour la BMP-2 bovine contenant la séquence d'acides aminés de la revendication 6.

8. Gène codant pour une protéine montrant des propriétés de la BMP-2 bovine et comprenant des séquences d'ADN :
(a) qui diffèrent d'une séquence d'ADN de la revendication 7 dans la séquence des codons du fait de la dégénérescence du code génétique ;
(b) qui s'hybrident avec une séquence d'ADN de la revendication 7 ou du paragraphe (a) ci-dessus ; ou
(c) représentent des fragments, des variations alléliques ou autres d'une séquence d'ADN de la revendication 7, que ces variations résultent de changements dans la séquence peptidique ou non.

9. Séquence d'ADN suivant la revendication 8, qui est une séquence d'ADN génomique.

10. Séquence d'ADN suivant la revendication 8, qui est une séquence d'ADNc.

11. Gène codant pour la BMP-4 humaine comprenant la séquence d'ADN suivante :

10 20 30 40 50 60 70
CTCTAGAGGG CAGAGGAGGA GGGAGGGAGG GAAGGAGCGC GGAGCCCGGC CCGGAAGCTA GGTGAGTGTG

80 90 100 110 120 130 140
GCATCCGAGC TGAGGGACGC GAGCCTGAGA CGCCGCTGCT GCTCCGGCTG AGTATCTAGC TTGTCTCCCC

150 160 170 180 190 200 210
GATGGGATTC CCGTCCAAGC TATCTCGAGC CTGCAGCGCC ACAGTCCCCG GCCCTCGCCC AGGTTCACTG

220 230 240 250 260 270 280
CAACCGTTCA GAGGTCCCCA GGAGCTGCTG CTGGCGAGCC CGCTACTGCA GGGACCTATG GAGCCATTCC

290 300 310 320 330 340 350
GTAGTGCCAT CCCGAGCAAC GCACTGCTGC AGCTTCCCTG AGCCTTTCCA GCAAGTTTGT TCAAGATTGG

360 370 380 390 400 (1)
CTGTCAAGAA TCATGGACTG TTATTATATG CCTTGTTTTC TGTCAAGACA CC ATG ATT CCT


417 432 447 462
GGT AAC CGA ATG CTG ATG GTC GTT TTA TTA TGC CAA GTC CTG CTA GGA GGC GCG


477 492 507
AGC CAT GCT AGT TTG ATA CCT GAG ACG GGG AAG AAA AAA GTC GCC GAG ATT CAG


522 537 552 567
GGC CAC GCG GGA GGA CGC CGC TCA GGG CAG AGC CAT GAG CTC CTG CGG GAC TTC


582 597 612 627
GAG GCG ACA CTT CTG CAG ATG TTT GGG CTG CGC CGC CGC CCG CAG CCT AGC AAG


642 657 672
AGT GCC GTC ATT CCG GAC TAC ATG CGG GAT CTT TAC CGG CTT CAG TCT GGG GAG


687 702 717 732
GAG GAG GAA GAG CAG ATC CAC AGC ACT GGT CTT GAG TAT CCT GAG CGC CCG GCC


747 762 777
AGC CGG GCC AAC ACC GTG AGG AGC TTC CAC CAC GAA GAA CAT CTG GAG AAC ATC


792 807 822 837
CCA GGG ACC AGT GAA AAC TCT GCT TTT CGT TTC CTC TTT AAC CTC AGC AGC ATC


852 867 882 897
CCT GAG AAC GAG GTG ATC TCC TCT GCA GAG CTT CGG CTC TTC CGG GAG CAG GTG


912 927 942
GAC CAG GGC CCT GAT TGG GAA AGG GGC TTC CAC CGT ATA AAC ATT TAT GAG GTT


957 972 987 1002
ATG AAG CCC CCA GCA GAA GTG GTG CCT GGG CAC CTC ATC ACA CGA CTA CTG GAC


1017 1032 1047
ACG AGA CTG GTC CAC CAC AAT GTG ACA CGG TGG GAA ACT TTT GAT GTG AGC CCT


1062 1077 1092 1107
GCG GTC CTT CGC TGG ACC CGG GAG AAG CAG CCA AAC TAT GGG CTA GCC ATT GAG


1122 1137 1152 1167
GTG ACT CAC CTC CAT CAG ACT CGG ACC CAC CAG GGC CAG CAT GTC AGG ATT AGC


1182 1197 1212
CGA TCG TTA CCT CAA GGG AGT GGG AAT TGG GCC CAG CTC CGG CCC CTC CTG GTC


1227 1242 1257 1272
ACC TTT GGC CAT GAT GGC CGG GGC CAT GCC TTG ACC CGA CGC CGG AGG GCC AAG


1287 1302 1317
CGT AGC CCT AAG CAT CAC TCA CAG CGG GCC AGG AAG AAG AAT AAG AAC TGC CGG


1332 1347 1362 1377
CGC CAC TCG CTC TAT GTG GAC TTC AGC GAT GTG GGC TGG AAT GAC TGG ATT GTG


1392 1407 1422 1437
GCC CCA CCA GGC TAC CAG GCC TTC TAC TGC CAT GGG GAC TGC CCC TTT CCA CTG


1452 1467 1482
GCT GAC CAC CTC AAC TCA ACC AAC CAT GCC ATT GTG CAG ACC CTG GTC AAT TCT


1497 1512 1527 1542
GTC AAT TCC AGT ATC CCC AAA GCC TGT TGT GTG CCC ACT GAA CTG AGT GCC ATC


1557 1572 1587
TCC ATG CTG TAC CTG GAT GAG TAT GAT AAG GTG GTA CTG AAA AAT TAT CAG GAG


1602 1617 (408) 1636 1646 1656
ATG GTA GTA GAG GGA TGT GGG TGC CGC TGAGATCAGG CAGTCCTTGA GGATAGACAG


1666 1676 1686 1696 1706 1716 1726
ATATACACAC CACACACACA CACCACATAC ACCACACACA CACGTTCCCA TCCACTCACC CACACACTAC

1736 1746 1756 1766 1776 1786 1796
ACAGACTGCT TCCTTATAGC TGGACTTTTA TTTAAAAAAA AAAAAAAAAA AATGGAAAAA ATCCCTAAAC

1806 1816 1826 1836 1846 1856 1866
ATTCACCTTG ACCTTATTTA TGACTTTACG TGCAAATGTT TTGACCATAT TGATCATATA TTTTGACAAA

1876 1886 1896 1906 1916 1926 1936
ATATATTTAT AACTACGTAT TAAAAGAAAA AAATAAAATG AGTCATTATT TTAAAAAAAA AAAAAAAACT

1946
CTAGAGTCGA CGGAATTC

12. Gène codant pour la BMP-4 humaine comportant la séquence d'acides aminés donnée à la revendication 11.

13. Gène codant pour une protéine montrant des propriétés de la BMP-4 et comprenant une séquence d'ADN :
(a) qui diffère d'une séquence d'ADN de la revendication 11 dans la séquence des codons du fait de la dégénérescence du code génétique ;
(b) qui s'hybride avec une séquence d'ADN de la revendication 11 ou du paragraphe (a) ci-dessus ; ou
(c) représente un fragment, une variation allélique ou autre d'une séquence d'ADN de la revendication 11, que cette variation résulte de changements dans la séquence peptidique ou non.

14. Séquence d'ADN suivant la revendication 13, qui est une séquence d'ADN génomique.

15. Séquence d'ADN suivant la revendication 13, qui est une séquence d'ADNc.

16. Vecteur contenant le gène ou la séquence d'ADN suivant l'une quelconque des revendications 1 à 15, en association active avec une séquence de contrôle d'expression.

17. Cellule transformée avec un vecteur de la revendication 16.

18. Cellule suivant la revendication 17, qui est une cellule mammifère, une cellule bactérienne, une cellule d'insecte ou une cellule de levure.

19. Cellule suivant la revendication 18, qui est une cellule CHO.

20. Protéine montrant des propriétés de la BMP-2, qui est codée par un gène ou une séquence d'ADN de l'une quelconque des revendications 1 à 10.

21. Protéine montrant des propriétés de la BMP-2, qui est obtenable par les étapes de culture dans un milieu de culture approprié d'une cellule transformée avec un vecteur d'expression comprenant un gène ou une séquence d'ADN de l'une quelconque des revendications 1 à 10, et de récupération de ladite protéine du milieu de culture précité.

22. Protéine montrant des propriétés de la BMP-4, qui est codée par un gène ou une séquence d'ADN de l'une quelconque des revendications 11 à 15.

23. Protéine montrant des propriétés de la BMP-4, qui est obtenable par les étapes de culture dans un milieu de culture approprié d'une cellule transformée avec un vecteur d'expression comprenant un gène ou une séquence d'ADN de l'une quelconque des revendications 11 à 15, et de récupération de ladite protéine du milieu de culture précité.

24. Procédé de production de la protéine suivant l'une ou l'autre des revendications 21 et 23, comprenant les étapes de culture dans un milieu de culture approprié de la cellule de la revendication 17 et d'isolement de ladite protéine du milieu de culture précité.

25. Composition pharmaceutique comprenant les protéines de l'une quelconque des revendications 20 à 23, individuellement ou en combinaison, et un véhicule pharmaceutiquement acceptable.

26. Composition pharmaceutique suivant la revendication 25, comprenant de plus une matrice pouvant distribuer la composition au site de l'anomalie osseuse ou cartilagineuse et formant une structure pour induire une formation osseuse ou cartilagineuse.

27. Composition pharmaceutique suivant la revendication 26, dans laquelle ladite matrice comprend de l'hydroxyapatite, du collagène, de l'acide polylactique ou du phosphate tricalcique.

28. Utilisation d'une protéine suivant l'une quelconque des revendications 20 à 23, individuellement ou en combinaison, pour la préparation d'une composition pharmaceutique pour induire une formation osseuse ou cartilagineuse.

## Revendications (Revendications pour l'(les) Etat(s) contractant(s) suivant(s): AT)

1. Procédé de préparation d'un gène codant pour la BMP-2 humaine comprenant la séquence d'ADN suivante : 
10         20         30         40         50         60         70
GTCGACTCTA GAGTGTGTGT CAGCACTTGG CTGGGGACTT CTTGAACTTG CAGGGAGAAT AACTTGCGCA

80         90         100        110        120        130        140
CCCCACTTTG CGCCGGTGCC TTTGCCCCAG CGGAGCCTGC TTCGCCATCT CCGAGCCCCA CCGCCCCTCC

150        160        170        180        190        200        210
ACTCCTCGGC CTTGCCCGAC ACTGAGACGC TGTTCCCAGC GTGAAAAGAG AGACTGCGCG GCCGGCACCC

220        230        240        250        260        270        280
GGGAGAAGGA GGAGGCAAAG AAAAGGAACG GACATTCGGT CCTTGCGCCA GGTCCTTTGA CCAGAGTTTT

290        300        310        320        330        340        350
TCCATGTGGA CGCTCTTTCA ATGGACGTGT CCCCGCGTGC TTCTTAGACG GACTGCGGTC TCCTAAAGGT

(1)                370                385                400
CGACC ATG GTG GCC GGG ACC CGC TGT CTT CTA GCG TTG CTG CTT CCC CAG GTC
      

415                430                445
CTC CTG GGC GGC GCG GCT GGC CTC GTT CCG GAG CTG GGC CGC AGG AAG TTC GCG


460                475                490                505
GCG GCG TCG TCG GGC CGC CCC TCA TCC CAG CCC TCT GAC GAG GTC CTG AGC GAG


520                535                550                565
TTC GAG TTG CGG CTG CTC AGC ATG TTC GGC CTG AAA CAG AGA CCC ACC CCC AGC


580                595                610
AGG GAC GCC GTG GTG CCC CCC TAC ATG CTA GAC CTG TAT CGC AGG CAC TCG GGT


625                640                655                670
CAG CCG GGC TCA CCC GCC CCA GAC CAC CGG TTG GAG AGG GCA GCC AGC CGA GCC


685                700                715
AAC ACT GTG CGC AGC TTC CAC CAT GAA GAA TCT TTG GAA GAA CTA CCA GAA ACG

 
730 745 760 775
AGT GGG AAA ACA ACC CGG AGA TTC TTC TTT AAT TTA AGT TCT ATC CCC ACG GAG


790 805 820 835
GAG TTT ATC ACC TCA GCA GAG CTT CAG GTT TTC CGA GAA CAG ATG CAA GAT GCT


850 865 880
TTA GGA AAC AAT AGC AGT TTC CAT CAC CGA ATT AAT ATT TAT GAA ATC ATA AAA


895 910 925 940
CCT GCA ACA GCC AAC TCG AAA TTC CCC GTG ACC AGT CTT TTG GAC ACC AGG TTG


955 970 985
GTG AAT CAG AAT GCA AGC AGG TGG GAA AGT TTT GAT GTC ACC CCC GCT GTG ATG


1000 1015 1030 1045
CGG TGG ACT GCA CAG GGA CAC GCC AAC CAT GGA TTC GTG GTG GAA GTG GCC CAC


1060 1075 1090 1105
TTG GAG GAG AAA CAA GGT GTC TCC AAG AGA CAT GTT AGG ATA AGC AGG TCT TTG


1120 1135 1150
CAC CAA GAT GAA CAC AGC TGG TCA CAG ATA AGG CCA TTG CTA GTA ACT TTT GGC


1165 1180 1195 1210
CAT GAT GGA AAA GGG CAT CCT CTC CAC AAA AGA GAA AAA CGT CAA GCC AAA CAC


1225 1240 1255
AAA CAG CGG AAA CGC CTT AAG TCC AGC TGT AAG AGA CAC CCT TTG TAC GTG GAC


1270 1285 1300 1315
TTC AGT GAC GTG GGG TGG AAT GAC TGG ATT GTG GCT CCC CCG GGG TAT CAC GCC


1330 1345 1360 1375
TTT TAC TGC CAC GGA GAA TGC CCT TTT CCT CTG GCT GAT CAT CTG AAC TCC ACT


1390 1405 1420
AAT CAT GCC ATT GTT CAG ACG TTG GTC AAC TCT GTT AAC TCT AAG ATT CCT AAG

 
1435                    1450                    1465                    1480
GCA TGC TGT GTC CCG ACA GAA CTC AGT GCT ATC TCG ATG CTG TAC CTT GAC GAG


        1495                    1510                    1525
AAT GAA AAG GTT GTA TTA AAG AAC TAT CAG GAC ATG GTT GTG GAG GGT TGT GGG


1540(396)    1553       1563       1573       1583       1593       1603
TGT CGC TAGTACAGCA AAATTAAATA CATAAATATA TATATATATA TATATTTTAG AAAAAAGAAA


AAAA ,
 dans lequel ledit procédé comprend les étapes suivantes
a) la sélection d'une bibliothèque de gènes construite à partir d'ADN ou d'ADNc provenant de U-2 OS avec un fragment de bBMP-2 marqué par hybridation,
b) l'isolement des clones positifs, et
c) l'isolement des inserts d'ADN de ces clones.

2. Procédé suivant la revendication 1, dans lequel le gène code pour la BMP-2 humaine ayant la séquence d'acides aminés donnée à la revendication 1.

3. Procédé de préparation d'un gène codant pour une protéine montrant des propriétés de la BMP-2 humaine et comprenant une séquence d'ADN :
a) qui diffère d'une séquence d'ADN de la revendication 1 dans la séquence des codons du fait de la dégénérescence du code génétique ;
b) qui s'hybride avec une séquence d'ADN de la revendication 1 ou du paragraphe (a) ci-dessus ; ou
c) représente un fragment, une variation allélique ou autre d'une séquence d'ADN de la revendication 1, que cette variation résulte de changements dans la séquence peptidique ou non,
dans lequel le procédé susdit comprend des techniques standards de biologie moléculaire.

4. Procédé suivant la revendication 3, dans lequel la séquence d'ADN est une séquence d'ADN génomique.

5. Procédé suivant la revendication 3, dans lequel la séquence d'ADN est une séquence d'ADNc.

6. Procédé de préparation d'un gène codant pour la BMP-2 bovine comprenant la séquence d'ADN suivante : 
(1)                15                30                45
GGC CAC GAT GGG AAA GGA CAC CCT CTC CAC AGA AGA GAA AAG CGG
G   H   D   G   K   G   H   P   L   H   R   R   E   K   R

                60                75                90
CAA GCA AAA CAC AAA CAG CGG AAA CGC CTC AAG TCC AGC TGT AAG
Q   A   K   H   K   Q   R   K   R   L   K   S   S   C   K

                105               120               135
AGA CAC CCT TTA TAT GTG GAC TTC AGT GAT GTG GGG TGG AAT GAC
R   H   P   L   Y   V   D   F   S   D   V   G   W   N   D

                150               165               180
TGG ATC GTT GCA CCG CCG GGG TAT CAT GCC TTT TAC TGC CAT GGG
W   I   V   A   P   P   G   Y   H   A   F   Y   C   H   G

                195               210               225
GAG TGC CCT TTT CCC CTG GCC GAT CAC CTT AAC TCC ACG AAT CAT
E   C   P   F   P   L   A   D   H   L   N   S   T   N   H

                240               255               270
GCC ATT CTC CAA ACT CTG GTC AAC TCA GTT AAC TCT AAG ATT CCC
A   I   V   Q   T   L   V   N   S   V   N   S   K   I   P

                385               300               315
AAG GCA TGC TGT GTC CCA ACA GAG CTC AGC GCC ATC TCC ATG CTG
K   A   C   C   V   P   T   E   L   S   A   I   S   M   L

                330               345               360
TAC CTT GAT GAG AAT GAG AAG GTG GTA TTA AAG AAC TAT CAG GAC
Y   L   D   E   N   E   K   V   V   L   K   N   Y   Q   D

                375               (129)       397       407
ATG GTT GTC GAG GGT TGT GGG TGT CGT TAGCACAGCA AAATAAAATA
M   V   V   E   G   C   G   C   R
 
417 427 437 447 457
TAAATATATA TATATATATA TTAGAAAAAC AGCAAAAAAA TCAAGTTGAC

467 477 487 497 507
ACTTTAATAT TTCCCAATGA AGACTTTATT TATGGAATGG AATGGAGAAA

517 527 537 547 557
AAGAAAAACA CAGCTATTTT GAAAACTATA TTTATATCTA CCGAAAAGAA

567 577 587
GTTGGGAAAA CAAATATTTT AATCAGAGAA TTATT,
 dans lequel ledit procédé comprend les étapes suivantes :
a) la sélection d'une bibliothèque de gènes construite à partir d'ADN ou d'ADNc provenant de foie bovin avec une sonde marquée conçue sur la base de la séquence d'acides aminés d'un fragment de bBMP-2,
b) l'isolement des clones positifs, et
c) l'isolement des inserts d'ADN de ces clones.

7. Procédé suivant la revendication 6, dans lequel le gène code pour de la BMP-2 bovine ayant la séquence d'acides aminés de la revendication 6.

8. Procédé de préparation d'un gène codant pour une protéine montrant des propriétés de la BMP-2 bovine et comprenant des séquences d'ADN :
a) qui diffèrent d'une séquence d'ADN de la revendication 7 dans la séquence des codons du fait de la dégénérescence du code génétique ;
b) qui s'hybrident avec une séquence d'ADN de la revendication 7 ou du paragraphe a) ci-dessus ; ou
c) représentent des fragments, des variations alléliques ou autres d'une séquence d'ADN de la revendication 7, que ces variations résultent de changements dans la séquence peptidique ou non,
dans lequel le procédé précité comprend des techniques standards de biologie moléculaire.

9. Procédé suivant la revendication 8, dans lequel la séquence d'ADN est une séquence d'ADN génomique.

10. Procédé suivant la revendication 8, dans lequel la séquence d'ADN est une séquence d'ADNc.

11. Procédé de préparation d'un gène codant pour la BMP-4 humaine comprenant la séquence d'ADN suivante : 
10 20 30 40 50 60 70
CTCTAGAGGG CAGAGGAGGA GGGAGGGAGG GAAGGAGCGC GGAGCCCGGC CCGGAAGCTA GGTGAGTGTG

80 90 100 110 120 130 140
GCATCCGAGC TGAGGGACGC GAGCCTGAGA CGCCGCTGCT GCTCCGGCTG AGTATCTAGC TTGTCTCCCC

150 160 170 180 190 200 210
GATGGGATTC CCGTCCAAGC TATCTCGAGC CTGCAGCGCC ACAGTCCCCG GCCCTCGCCC AGGTTCACTG

220 230 240 250 260 270 280
CAACCGTTCA GAGGTCCCCA GGAGCTGCTG CTGGCGAGCC CGCTACTGCA GGGACCTATG GAGCCATTCC

290 300 310 320 330 340 350
GTAGTGCCAT CCCGAGCAAC GCACTGCTGC AGCTTCCCTG AGCCTTTCCA GCAAGTTTGT TCAAGATTGG

360 370 380 390 400 (1)
CTGTCAAGAA TCATGGACTG TTATTATATG CCTTGTTTTC TGTCAAGACA CC ATG ATT CCT
...

417 432 447 462
GGT AAC CGA ATG CTG ATG GTC GTT TTA TTA TGC CAA GTC CTG CTA GGA GGC GCG


477 492 507
AGC CAT GCT AGT TTG ATA CCT GAG ACG GGG AAG AAA AAA GTC GCC GAG ATT CAG


522 537 552 567
GGC CAC GCG GGA GGA CGC CGC TCA GGG CAG AGC CAT GAG CTC CTG CGG GAC TTC


582 597 612 627
GAG GCG ACA CTT CTG CAG ATG TTT GGG CTG CGC CGC CGC CCG CAG CCT AGC AAG


642 657 672
AGT GCC GTC ATT CCG GAC TAC ATG CGG GAT CTT TAC CGG CTT CAG TCT GGG GAG


687 702 717 732
GAG GAG GAA GAG CAG ATC CAC AGC ACT GGT CTT GAG TAT CCT GAG CGC CCG GCC

 
747 762 777
AGC CGG GCC AAC ACC GTG AGG AGC TTC CAC CAC GAA GAA CAT CTG GAG AAC ATC


792 807 822 837
CCA GGG ACC AGT GAA AAC TCT GCT TTT CGT TTC CTC TTT AAC CTC AGC AGC ATC


852 867 882 897
CCT GAG AAC GAG GTG ATC TCC TCT GCA GAG CTT CGG CTC TTC CGG GAG CAG GTG


912 927 942
GAC CAG GGC CCT GAT TGG GAA AGG GGC TTC CAC CGT ATA AAC ATT TAT GAG GTT


957 972 987 1002
ATG AAG CCC CCA GCA GAA GTG GTG CCT GGG CAC CTC ATC ACA CGA CTA CTG GAC


1017 1032 1047
ACG AGA CTG GTC CAC CAC AAT GTG ACA CGG TGG GAA ACT TTT GAT GTG AGC CCT


1062 1077 1092 1107
GCG GTC CTT CGC TGG ACC CGG GAG AAG CAG CCA AAC TAT GGG CTA GCC ATT GAG


1122 1137 1152 1167
GTG ACT CAC CTC CAT CAG ACT CGG ACC CAC CAG GGC CAG CAT GTC AGG ATT AGC


1182 1197 1212
CGA TCG TTA CCT CAA GGG AGT GGG AAT TGG GCC CAG CTC CGG CCC CTC CTG GTC


1227 1242 1257 1272
ACC TTT GGC CAT GAT GGC CGG GGC CAT GCC TTG ACC CGA CGC CGG AGG GCC AAG


1287 1302 1317
CGT AGC CCT AAG CAT CAC TCA CAG CGG GCC AGG AAG AAG AAT AAG AAC TGC CGG


1332 1347 1362 1377
CGC CAC TCG CTC TAT GTG GAC TTC AGC GAT GTG GGC TGG AAT GAC TGG ATT GTG


1392 1407 1422 1437
GCC CCA CCA GGC TAC CAG GCC TTC TAC TGC CAT GGG GAC TGC CCC TTT CCA CTG


1452 1467 1482
GCT GAC CAC CTC AAC TCA ACC AAC CAT GCC ATT GTG CAG ACC CTG GTC AAT TCT

 
1497 1512 1527 1542
GTC AAT TCC AGT ATC CCC AAA GCC TGT TGT GTG CCC ACT GAA CTG AGT GCC ATC


1557 1572 1587
TCC ATG CTG TAC CTG GAT GAG TAT GAT AAG GTG GTA CTG AAA AAT TAT CAG GAG


1602 1617 (408) 1636 1646 1656
ATG GTA GTA GAG GGA TGT GGG TGC CGC TGAGATCAGG CAGTCCTTGA GGATAGACAG


1666 1676 1686 1696 1706 1716 1726
ATATACACAC CACACACACA CACCACATAC ACCACACACA CACGTTCCCA TCCACTCACC CACACACTAC

1736 1746 1756 1766 1776 1786 1796
ACAGACTGCT TCCTTATAGC TGGACTTTTA TTTAAAAAAA AAAAAAAAAA AATGGAAAAA ATCCCTAAAC

1806 1816 1826 1836 1846 1856 1866
ATTCACCTTG ACCTTATTTA TGACTTTACG TGCAAATGTT TTGACCATAT TGATCATATA TTTTGACAAA

1876 1886 1896 1906 1916 1926 1936
ATATATTTAT AACTACGTAT TAAAAGAAAA AAATAAAATG AGTCATTATT TTAAAAAAAA AAAAAAAACT

1946
CTAGAGTCGA CGGAATTC,
 dans lequel le procédé précité comprend les étapes suivantes :
a) la sélection d'une bibliothèque de gènes construite à partir d'ADN ou d'ADNc provenant d'U-2 OS avec un fragment bBMP-2 marqué par hybridation,
b) l'isolement des clones positifs, et
c) l'isolement des inserts d'ADN de ces clones.

12. Procédé suivant la revendication 11, dans lequel le gène code pour la BMP-4 humaine ayant la séquence d'acides aminés donnée à la revendication 11.

13. Procédé de préparation d'un gène codant pour une protéine montrant des propriétés de la BMP-4 et comprenant une séquence d'ADN :
a) qui diffère d'une séquence d'ADN de la revendication 11 dans la séquence des codons du fait de la dégénérescence du code génétique ;
b) qui s'hybride avec une séquence d'ADN de la revendication 11 ou du paragraphe a) ci-dessus ; ou
c) représente un fragment, une variation allélique ou autre d'une séquence d'ADN de la revendication 11, que cette variation résulte de changements dans la séquence peptidique ou non,
dans lequel le procédé précité comprend des techniques standards de biologie moléculaire.

14. Procédé suivant la revendication 13, dans lequel la séquence d'ADN est une séquence d'ADN génomique.

15. Procédé suivant la revendication 13, dans lequel la séquence d'ADN est une séquence d'ADNc.

16. Vecteur contenant le gène ou la séquence d'ADN préparé suivant l'une quelconque des revendications 1 à 15, en association active avec une séquence de contrôle d'expression.

17. Cellule transformée avec un vecteur de la revendication 16.

18. Cellule suivant la revendication 17, qui est une cellule mammifère, une cellule bactérienne, une cellule d'insecte ou une cellule de levure.

19. Cellule suivant la revendication 18, qui est une cellule CHO.

20. Procédé de préparation d'une protéine montrant des propriétés de la BMP-2, dans lequel ledit procédé comprend les étapes de culture dans un milieu de culture approprié d'une cellule transformée avec un vecteur d'expression comprenant un gène ou une séquence d'ADN préparé suivant l'une quelconque des revendications 1 à 10 et de récupération de ladite protéine du milieu de culture précité.

21. Procédé de préparation d'une protéine montrant des propriétés de la BMP-4, dans lequel ledit procédé comprend les étapes de culture dans un milieu de culture approprié d'une cellule transformée avec un vecteur d'expression comprenant un gène ou une séquence d'ADN préparé suivant l'une quelconque des revendications 11 à 15 et de récupération de ladite protéine du milieu de culture précité.

22. Procédé de production d'une protéine montrant des propriétés de la BMP-2 ou BMP-4, comprenant les étapes de culture dans un milieu de culture approprié de la cellule de la revendication 17 et d'isolement de ladite protéine du milieu de culture précité.

23. Procédé de préparation d'une composition pharmaceutique comprenant la combinaison des protéines préparées suivant l'une quelconque des revendications 20 à 22, individuellement ou en combinaison avec un véhicule pharmaceutiquement acceptable.

24. Procédé suivant la revendication 23, dans lequel la composition pharmaceutique susdite comprend de plus une matrice pouvant distribuer la composition au site de l'anomalie osseuse ou cartilagineuse et constituer une structure pour induire une formation osseuse ou cartilagineuse.

25. Procédé suivant la revendication 24, dans lequel la matrice comprend de l'hydroxyapatite, du collagène, de l'acide polylactique ou du phosphate tricalcique.

26. Utilisation d'une protéine préparée suivant l'une quelconque des revendications 20 à 22, individuellement ou en combinaison, pour la préparation d'une composition pharmaceutique pour induire une formation osseuse ou cartilagineuse.
